(19) Europäisches Patentamt
European Patent Office
Office européen des brevets

(11) Publication number: **0 242 957 B1**

(12) # EUROPEAN PATENT SPECIFICATION

(45) Date of publication of the patent specification: **12.09.90**

(51) Int. Cl.[5]: **C07D 241/18**, C07D 403/06, C07D 409/12, A61K 31/495

(21) Application number: **87301680.2**

(22) Date of filing: **25.02.87**

(54) **1-Substituted Alkyl-1, 2-Dihydro-2-Pyrazinone derivatives.**

(30) Priority: **25.02.86 JP 38210/86**

(43) Date of publication of application: **28.10.87 Bulletin 87/44**

(45) Publication of the grant of the patent: **12.09.90 Bulletin 90/37**

(84) Designated Contracting States: **CH DE FR GB IT LI**

(56) References cited:
**EP-A- 0 072 932**
**EP-A- 0 096 517**
**GB-A- 1 600 127**
**US-A- 3 935 214**
**US-A- 4 181 724**

(73) Proprietor: **TOYO JOZO KABUSHIKI KAISHA, 632-1 Mifuku Ohito-cho, Tagata-gun Shizuoka-ken(JP)**

(72) Inventor: **Yaso, Masao, 848-1, Takyo Ohito-cho, Tagata-gun Shizuoka-ken(JP)**
Inventor: **Suzuki, Yukio, 525-1, Mifuku Ohito-cho, Tagata-gun Shizuoka-ken(JP)**
Inventor: **Shibata, Kensuke, 846-3, Nanjyo Nirayama-cho, Tagata-gun Shizuoka-ken(JP)**
Inventor: **Hayashi, Eiichi, 7-32, Midori-cho, Shizuoka-shi Shizuoka-ken(JP)**

(74) Representative: **Myerscough, Philip Boyd et al, J.A.Kemp & Co. 14, South Square Gray's Inn, London, WC1R 5EU(GB)**

EP 0 242 957 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

This invention relates to novel 1-substituted alkyl-2-dihydro pyrazine derivatives and 1-substituted alkyl-2-hexahydro quinoxaline derivatives, which are pharmaceuticals useful for improvement in circulation and metabolic disorder having activities of platelet aggregation inhibition, vasodilation and/or anti-lipoperoxide generation.

Recently, not a little number of compounds having platelet aggregation inhibitory activities have been reported. Amongst these, however, only compounds having pyrazine ring as the main structural element such as tetramethyl pyrazine (16th Heterocyclic chemistry Symposium (Osaka) p. 65-68 (1984) and a 2-higher-fatty acid acyloxymethyl pyrazine (Japan. Pat. Unexam. Publ. No. 59-219269) have been known.

It is of great importance to find excellent pharmaceuticals having stronger inhibitory activities for platelet aggregation in order to use these in the therapy of circulation and metabolic disorders.

We have found that 1-substituted alkyl-2-oxo-1,2-dihydropyrazine derivatives and 1-substituted alkyl-2-oxo-1,2,5,6,7,8-hexahydroquinoxaline derivatives have an inhibitory action on platelet aggregation, vasodilation activity and/or anti-lipoperoxide generation, and are thus expected to have an excellent pharmaceutical action.

The present invention provides a compound of the formula (1)

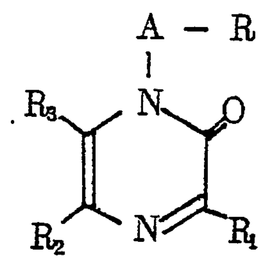

(1)

wherein R is hydroxyl, halogen $C_{1-6}$ alkanoyloxy, $R_4$-carbamoyloxy, arylthio, 1-methyltetrazole-5-yl-thio, 1-imidazolyl, morpholino,

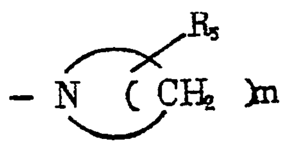

,

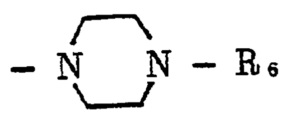

,

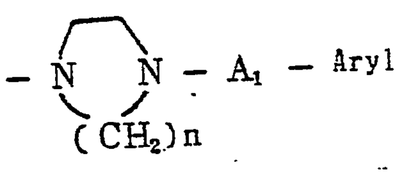

or

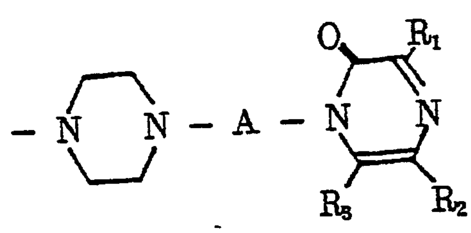

in which $R_4$ is lower $C_{1-6}$ alkyl or aryl, $R_5$ is hydrogen, $C_{1-6}$ alkyl or aryl, $R_6$ is hydrogen, $C_{1-4}$ alkyl, hydroxy $C_{1-4}$ alkyl, aryl, aryl-$C_{2-4}$ alkanoyl, arylcarbonyl, arylsulfonyl or thienyl $C_{2-4}$ alkanoyl, A and $A_1$ are $C_{1-3}$ alkylene, m is an integer of from 4 to 6, n is the integer of 2 or 3, $R_1$ is alkyl, aryl-$C_{1-2}$ alkyl or aryl; and $R_2$ and $R_3$ are each $C_{1-3}$ alkyl or together constitute tetramethylene; and pharmacologically acceptable non toxic salts thereof; wherein aryl means phenyl unsubstituted or substituted by $C_{1-3}$ alkyl, halogen, nitro or lower alkoxy.

Examples of salts are salt of inorganic acids such as hydrochloride, sulfate and phosphate, and organic acids such as hydrochloride, sulfate and phosphate, and organic acids such as acetate, propionate, butyrate, glycolate, gluconate, malate, tartrate, succinate, mandelate, aspartate, glutarate, methane-sulfonate and toluenesulfonate. Other known salts can be included.

The compound of formula (1) can be produced by the following processes.

(A) A compound of formula (1) or a pharmacologically acceptable non-toxic salt thereof wherein R is hydroxyl of the formula (1a)

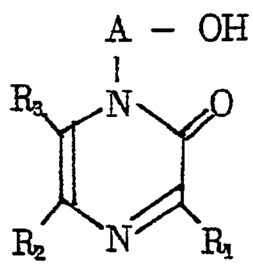

[1a]

wherein $R_1$, $R_2$, $R_3$ and A are as defined above can be produced by reacting a compound of formula (2)

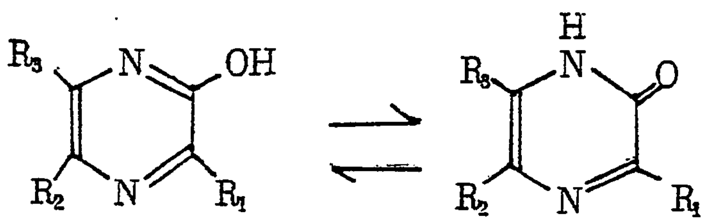

(2)

wherein $R_1$, $R_2$ and $R_3$ are as defined above, with a hydroxyalkyl halide of the formula

X–A–OH

wherein X is a halogen and A is as defined above, in an organic solvent and, if desired, converting the resultant compound of formula (1a) into a pharmacologically acceptable non-toxic salt thereof.

In the compound of formula (2), $R_1$ is alkyl, aryl or aryl-$C_{1-3}$ alkyl, wherein aryl is as defined above. The alkyl is preferably a saturated or unsaturated $C_{1-20}$ alkyl and may optionally be branched.

Examples are methyl, ethyl, propyl, isopropyl, butyl, isobutyl, sec-butyl, t-butyl, pentyl, isopentyl, hexyl, heptyl, octyl, nonyl, decylc, undecyl, docecyl, tridecyl, tetradecyl, pentadecyl and hexadecyl. Examples of the $C_{1-3}$ alkyl moiety in the aryl-$C_{1-3}$ alkyl groupe are methyl, 2-ethyl, 1-ethyl, 3-propyl and 1-propyl. Examples thereof of the aryl-$C_{1-3}$ alkyl group are benzyl, p-chlorobenzyl, 2-phenylethyl and 1-phenyl-ethyl.

In the compound of formula (2), $R_2$ and $R_3$ are $C_{1-3}$ alkyl, or together constitute tetramethylene. The $C_{1-3}$ alkyl can be methyl, ethyl or propyl. The compound of formula (2) wherein $R_2$ and $R_3$ together constitute tetramethylene, can be designated as 2-hydroxy-3-substituted-hexahydro quinoxaline. Among the compounds of formula (2), especially in which $R_2$ and $R_3$ are $C_{1-3}$ alkyl, i.e. 2-hydroxy-3-substituted-5,6-di $C_{1-3}$ alkylpyrazine, some compounds are known. However, of course, unknown compounds are included. These compounds can be produced by the process or its improved method disclosed in J. Am. Chem. Soc., 71: 78–81 (1949) and ibid. 74: 1580–1583 (1952). Novel compounds can also be produced according to the methods described in the above references. The compounds of formula (2) wherein $R_2$ and $R_3$ together constitute tetramethylene, i.e. 2-hydroxy-3-substituted-hexahydro quinoxaline derivatives, can be produced by the process found by the present inventors, which comprises reacting an α-amino acidamide except glycine with 1,2-cyclohexanedione in an alkaline medium.

The group A in the hydroxylalkyl halide is $C_{1-3}$ alkylene, that is methylene, ethylene, methylmethylene, propylene, 1-methylethylene, 1,1-dimethylmethylene or 1-ethylmethylene; Ethylene is preferred.

The group X in the hydroxyalkyl halide is a halogen such as chlorine or bromine; in general chlorine is preferred.

The preferred compound is ethylene chlorohydrine.

The reaction of the compound of formula (2), with the hydroxyalkyl halide proceeds in an organic solvent such as butanol.

The above reaction proceeds preferably in the presence of an aqueous alkali such as a hydroxy group containing alkali, and preferably if possible under heating. Isolation of the product of formula (1a) can be performed by adding water to the reaction mixture and extracting with a water immiscible organic solvent.

(B) A compound of formula (1), wherein R is $C_{1-6}$ alkanoyloxy (herein-after designated as a compound of formula (1b)) or a pharmacologically acceptable non-toxic salt thereof can be produced by acylating the compound of formula (1a) to convert the hydroxyl group thereof into a $C_{1-6}$ alkanoyloxy group, preferably with a $C_{1-6}$ fatty acid or a reactive derivative thereof, and, if desired, converting the resultant

compound of formula (16) into a pharmacologically acceptable non-toxic salt thereof.

Examples of the $C_{1-6}$ fatty acid, which is optionally branched, are acetic acid, propionic acid, butyric acid, isobutyric acid, sec-butyric acid, valeric acid, isovaleric acid and hexanoic acid. Examples of reactive derivatives are known acylating agents for hydroxyl, such as acid halide, acid anhydride, mixed anhydride or active ester. A $C_{1-6}$ fatty acid per se can be acylated in the presence of a condensation reagent such as N,N-dicyclohexyl carbodiimide (DCC).

In the above reactions, co-produced acid can be removed by an acid-binder, to example a known tertiary organic amine such as pyridine or triethylamine.

The product of formula (1b) can be isolated by pouring the reaction mixture into diluted aqueous alkali and extracting with a water immiscible organic solvent.

(C) A compound of formula (1) wherein R is $R_4$-carbamoyloxy (herein-after designated as a compound of formula (1c), or a pharmacologically acceptable non-toxic salt thereof, can be produced by reacting the compound of formula (1a) with a $C_{1-6}$ alkyl isocyanate or aryl isocyanate and, if desired, converting the resultant compound of formula (1c) into a pharmacologically acceptable non-toxic salt thereof.

The $C_{1-6}$ alkyl carbamoyloxy is optionally branched.

The reaction of the compound of formula (1a) and the isocyanate compound proceeds in an organic solvent such as pyridine. Isolation of the product of formula (1c) can be made by removing the solvent from the reaction mixture and extracting the residue with an inert organic solvent in an aqueous medium, or precipitating the product of formula (1c) by adding a solvent such as an ether.

(D) A compound of formula (1) or a pharmacologically acceptable non-toxic salt thereof wherein R is halogen, of the formula (1d)

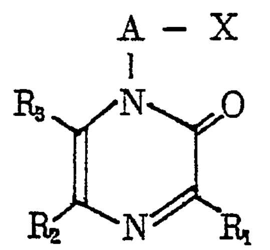

[1d]

wherein X is halogen, and A, $R_1$, $R_2$ and $R_3$ are as defined above, can be produced by halogenating a compound of formula (1a) with halogenating agent, and, if desired, converting the resultant compound of formula (1d) into a pharmacologically acceptable non-toxic salt thereof.

Examples of the halogenating agent are known halogenating agents. The product of formula (1d) can be used to prepare compounds of formulae (1e) and (1f) hereinbelow. In the reaction, known chlorination or bromination reagents can be used. Conventional chlorination reagents such as $SOCl_2$, $PCl_5$ or $POCl_3$ can be applied. Halogenation reactions are made, in general, in an inert organic solvent such as chloroform. Reaction proceeds at room temperature. Isolation of the product of formula (1d) can be made by adding a water immiscible organic solvent such as chloroform, washing with diluted aqueous alkali, dehydrating the organic layer and removing the solvent therefrom.

The compound of formula (1d) can be used without purifying, such as by silica-gel column chromatography, when used to prepare compounds of formulae (1e) and (1f).

(E) A compound of formula (1) wherein R is arylthio or 1-methyltetrazole-5-yl-thio (hereinafter designated as a compound of formula (1e), or a pharmacologically acceptable non-toxic salt thereof, can be produced by reacting the compound of formula (1d) with an alkali or alkaline earth metal salt of arylmercaptan or 1-methyl-5-mercaptotetrazole in an organic solvent and, if desired, converting the resultant compound of formula (1e) into a pharmacologically acceptable non-toxic salt thereof.

The aryl in the arylthio is as previously defined.

Example of the alkali or alkaline earth salts of arylmercaptan or 1-methyl-5-mercaptotetrazole are sodium and potassium salts.

The reaction of the compound of formula (1d) and the salt of the mercapto compound proceeds, in general, in an organic solvent such as dimethyl formamide (DMF).

Isolation of the product of formula (1e) can be made by removing the reaction solvent and extracting the residue with a water immiscible organic solvent in the presence of diluted aqueous alkali. (F) A compound of formula (1) wherein R is not hydroxyl, halogen, $C_{1-6}$ alkanoyloxy, $R_4$-carbamoyloxy, arylthio or 1-methyltetrazole-5-yl,thio (hereinafter designated as a compound of formula (1f), or a pharmacologically acceptable non-toxic salt thereof can be produced by reacting the compound of formula (1d) with an amine of the formula

R'–H

wherein R' is 1-imidazolyl, morpholino,

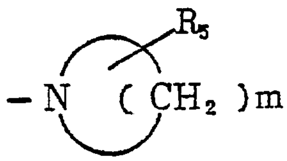 , 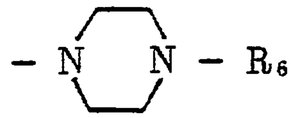 , 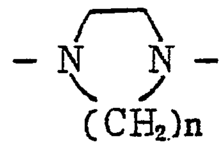

$A_1$ -aryl or 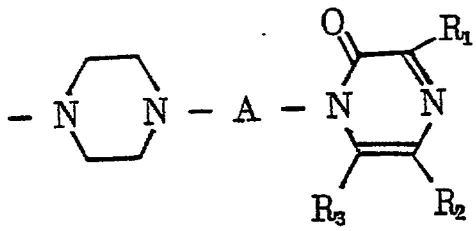

wherein $R_1$, $R_2$, $R_3$, $R_5$, $R_6$, A, $A_1$, m and n are as defined above and, if desired, converting the resultant compound of formula (1f) into a pharmacologically acceptable non-toxic salt thereof.

The ring 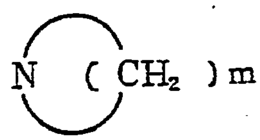

in the above 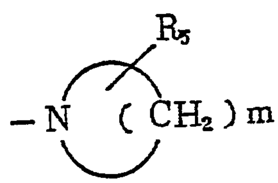

is a pyrrolidine, piperidine or hexamethyleneimine ring. $R_5$ is hydrogen, $C_{1-6}$ alkyl or aryl as defined above. The position of the $R_5$ group may be anywhere in the ring except at a position 1. The alkyl may be branched.

The group $R_6$ in

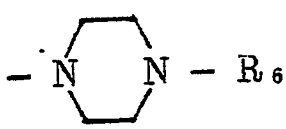

is hydrogen, $C_{1-4}$ alkyl, hydroxy-$C_{1-4}$ alkyl, aryl, aryl-$C_{2-4}$ alkanoyl, arylcarbonyl, arylsulfonyl or thienyl-$C_{2-4}$ alkanoyl. Examples of the alkyl, which may optionally be branched, are methyl, 2-ethyl, 1-ethyl, propyl, isopropyl and 1-methylethyl. The aryl groups are phenyl, which may optionally be substituted by $C_{1-3}$ alkyl halogen, nitro or lower alkoxy, such as phenyl, tolyl, xylyl, p(m or o)-nitrophenyl, p(m or o)-fluorophenyl, 2,4(2,3–, 3,4– 2,5–, 3,5– or 2,6–)-dichlorophenyl, p(m or o)-nitrophenyl, p(m or o)-methoxyphenyl, and 2,3(2,4–, 3,4–2,5–, 3,5– or 2,6–)–dimethoxyphenyl. The $C_{2-4}$ alkanoyl may optionally be branched, and may be acetyl, propionyl, butyryl, isobutyryl or sec-butyryl.

The ring in

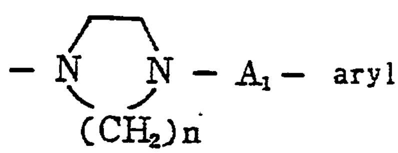

wherein n is 2 or 3 is a piperazine or homopiperazine ring. $A_1$ is a $C_{1-3}$ alkylene such as methylene, ethylene, methylmethylene, propylene, 1-methyl-ethylene or 1-ethylmethylene, preferably methylene or ethylene. The aryl is phenyl which may optionally be substituted by $C_{1-3}$ alkyl, halogen, nitro or lower alkoxy. Examples thereof have been hereinbefore illustrated.

Examples of the amine are 1-imidazole, morpholine, pyrrolidine, piperidine, 4-methylpiperazine, 4-ethylpiperazine, 4-butylpiperazine, 4-(2-hydroxyethyl)-piperazine, 4-phenyl-piperazine, 4-(o-chlorophenyl)-piperazine, 4-(o-methoxyphenyl)-piperazine, 4-(phenylacetyl)-piperazine, 4-benzoyl-piperazine, 4-(p-chlorobenzoyl)-piperazine, 4-(p-nitrobenzoyl)-piperazine, 4-(p-methoxybenzoyl)-piperazine, 4-(2,3-dimethoxybenzoyl)-piperazine, 4-(2,4-dimethoxybenzoyl)-piperazine, 4-(3,4-dimethoxybenzoyl)-piperazine, 4-(3,4,5-trimethoxybenzoyl)-piperazine, 4-benzenesulfonyl-piperazine, 4-(p-chlorobenzene-sulfonyl)-piperazine, 4-(2-thienylacetyl)-piperazine, 4-benzyl-piperazine, 4-(p-methylbenzyl)-piperazine, 4-(p-chlorobenzyl )-piperazine, 4-(2,4-dichlorobenzyl)-piperazine, 4-(p-fluorobenzyl)-piperazine, 4-(p-nitrobenzyl)-piperazine, 4-(p-methoxybenzyl)-piperazine, 4-benzyl-homopiperazine, 4-(p-methylbenzyl)-homopiperazine, 4-(p-chlorobenzyl)-homopiperazine, 4-(p-fluorobenzyl)-homopiperazine, 4-(p-nitrobenzyl)-homopiperazine and 4-(p-methoxybenzyl)-homopiperazine.

The reaction of the compound of formula (1d) and the amine can conventionally proceed in an inert organic solvent such as benzene under heating. In the reaction hydrogen halogenide is generated so that the reaction proceeds in the presence of an acid binder, for example a known tertiary organic amine such as triethylamine. The compound of formula (1f) can be isolated by pouring the reaction mixture into diluted aqueous alkali and extracting with a water immiscible organic solvent such as benzene or chloroform.

(G) A compound of formula (1) wherein R is

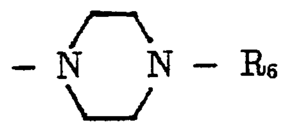 ,

in which $R_6$ is aryl-$C_{2-4}$-alkanoyl, arylcarbonyl, arylsulfonyl or thienyl-$C_{2-4}$ alkanoyl (hereinafter designated as a compound of formula (1g), or a pharmacologically acceptable non-toxic salt thereof can be produced by the process (F) but can also be produced by acylating a compound of formula (1f')

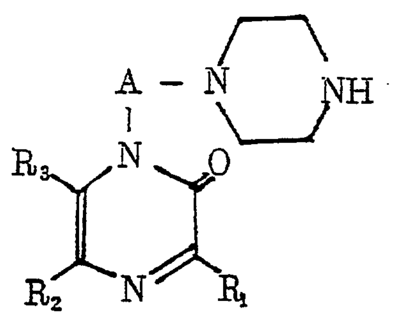

[1f']

wherein $R_1$, $R_2$, $R_3$ and A are as defined above, such as to convert the piperazinyl group thereof into a group of formula

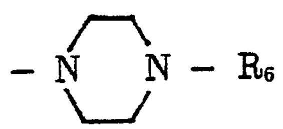

and, if desired, converting the resultant compound of formula (1g) into a pharmacologically acceptable non-toxic salt thereof.

The acylating agent may be an organic acid or reactive derivative thereof, for example an aryl-$C_{1-4}$ fatty acid, aryl-carboxylic acid, arylsulfonic acid or thienyl-$C_{2-4}$ fatty acid, in which aryl is phenyl optionally substituted by $C_{1-3}$ alkyl, halogen, nitro or lower alkoxy, and the $C_{2-4}$ fatty acid is optionally branched and in, for example, acetic acid, propionic acid, butyric acid, isobutyric acid or sec-butyric acid. Examples of organic acids are phenyl acetic acid, benzoic acid, p-chlorobenzoic acid, p-nitrobenzoic acid, p-methoxybenzoic acid, 2,3-dimethoxy benzoic acid, 2,4-dimethoxybenzoic acid, 3,4-dimethoxybenzoic acid, 3,4,5-trimethoxybenzoic acid, benzenesulfonic acid, p-toluenesulfonic acid and 2-thienylacetic acid. Examples of reactive derivatives are known acylating agents for amino groups, for example an acid halide, acid anhydride, mixed anhydride or activated ester. An organic acid per se can be acylated in the presence of a condensation agent such as DCC. In the reaction acid is coproduced, and thus the reaction may proceed in the presence of an acid binder, for example a known tertiary organic amine such as pyridine or triethylamine. The acylating reaction generally proceeds in a conventional organic solvent. The compound of formula (1g) can be isolated by pouring the reaction mixture into diluted aqueous alkali and extracting with a water immiscible organic solvent.

The thus obtained compound of formula (1) is purified, if required, by column chromatography using silica-gel, activated alumina or an adsorption resin with an elution solvent such as chloroform-methanol or benzene-ethyl acetate.

The compound of formula (1) is in general produced in the form of a free base, but it can be produced in the form of a conventional salt thereof. For example, a hydrochloride can be prepared by dissolving the compound of formula (1) in methanol or ethanol, adding a slightly excess-molar amount of hydrochloric acid, and isolating the precipitated material, or if not precipitated, by adding ether thereto to precipitate. The molar ratio of hydrochloric acid may be altered according to the nature of the compound of formula (1).

Examples of the compound of formula (1) of the present invention are illustrated in Table I.

The present invention also provides a pharmaceutical composition comprising as active ingredient a compound of formula (1) or a pharmacologically acceptable non toxic salt thereof, together with a pharmaceutically acceptable carrier or diluent.

**Table 1**

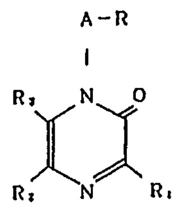

Me ; methyl
Et ; ethyl
Pro ; propyl
Iso-Pro ; isopropyl
Bu ; buthyl
Iso-Bu ; isobuthyl
sec-Bu ; sec-buthyl

Ph ; benzene ring
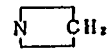 ; piperidine ring 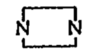 ; pioerazine
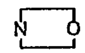 ; morpholine ring 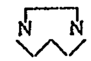 ; homopiperazine

( ) Figure and number in ( ) mean a position of substituent

| Compound No. | $R_1$ | $R_2$ | $R_3$ | A | R |
|---|---|---|---|---|---|
| 018 | Me | Me | Me | $-CH_2CH_2-$ | 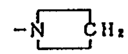 |
| 019 | Me | Me | Me | 〃 | 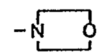 |
| 020 | Me | Me | Me | 〃 | 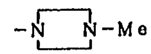 $-Me$ |
| 021 | Me | Me | Me | 〃 | 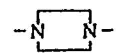 $-CH_2CH_2-OH$ |
| 022 | Me | Me | Me | 〃 | 1-imidazolyl |
| 023 | Me | Me | Me | 〃 | 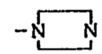 $-CH_2Ph$ |
| 024 | Me | Me | Me | 〃 | 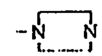 $-CH_2Ph-Cl$ (p) |
| 025 | Me | Me | Me | 〃 | 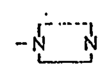 $-CH_2Ph-Cl$ (o) |
| 026 | Me | Me | Me | 〃 | $-OH$ |
| 027 | Me | Me | Me | 〃 | $-S-Ph$ |
| 028 | Me | Me | Me | 〃 | $-S-$ (1-methyltetrazole-5-yl) |

| Compound No. | $R_1$ | $R_2$ | $R_3$ | A | R |
|---|---|---|---|---|---|
| 061 | Me | Me | Me | $-CH_2CH_2-$ | $-O-CO-Me$ |
| 062 | Me | Me | Me | 〃 | $-O-CONH-Me$ |
| 063 | Me | Me | Me | 〃 | $-O-CONH-Ph$ |
| 064 | Me | Me | Me | 〃 | $-O-CONH-Ph-Cl$ (p) |
| 101 | Me | Me | Me | 〃 | 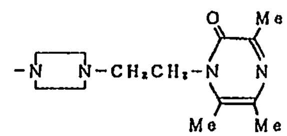 |
| 109 | Ph | Me | Me | 〃 | $-OH$ |
| 110 | Ph | Me | Me | 〃 | 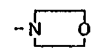 |
| 111 | Ph | Me | Me | 〃 | 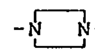 $-CH_2Ph-Cl$ (p) |
| 112 | $CH_2$ ph | Me | Me | 〃 | $-OH$ |
| 113 | 〃 | Me | Me | 〃 | 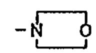 |
| 114 | 〃 | Me | Me | 〃 | 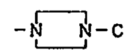 $-CH_2Ph$ |
| 115 | 〃 | Me | Me | 〃 | 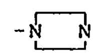 $-CH_2Ph-Cl$ (p) |
| 116 | 〃 | Me | Me | 〃 | 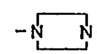 $-CH_2Ph-Cl$ (o) |
| 117 | Pro | $-(CH_2)_4-$ | | 〃 | 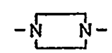 $-CH_2CH_2-OH$ |
| 118 | Pro | 〃 | | 〃 | 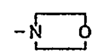 |
| 119 | Pro | 〃 | | 〃 | 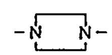 $-CH_2Ph$ |

| Compound No. | $R_1$ | $R_2$ | $R_3$ | A | R |
|---|---|---|---|---|---|
| 120 | Pro | $-(CH_2)_4-$ | | $-CH_2CH_2-$ | 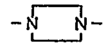 $-CH_2Ph-Cl$ (p) |
| 121 | Pro | Me | Me | 〃 | 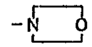 |
| 122 | Pro | Me | Me | 〃 | 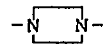 $-CH_2Ph$ |
| 123 | Iso-Bu | Me | Me | 〃 | 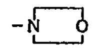 |
| 124 | 〃 | Me | Me | 〃 | 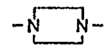 $-CH_2Ph$ |
| 125 | 〃 | Me | Me | 〃 | 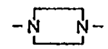 $-CH_2Ph-Cl$ (p) |
| 126 | 〃 | Me | Me | 〃 | 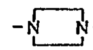 $-CH_2Ph-Cl$ (m) |
| 127 | 〃 | $-(CH_2)_4-$ | | 〃 | 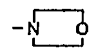 |
| 128 | 〃 | 〃 | | 〃 | 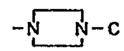 $-CH_2Ph$ |
| 129 | 〃 | 〃 | | 〃 | 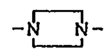 $-CH_2Ph-Cl$ (m) |
| 130 | Pro | Et | Et | 〃 | 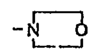 |
| 131 | Pro | Et | Et | 〃 | 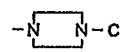 $-CH_2Ph$ |
| 132 | Pro | Et | Et | 〃 | 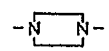 $-CH_2Ph-Cl$ (p) |
| 133 | Pro | Et | Et | 〃 | 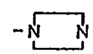 $-CH_2Ph-Cl$ (m) |
| 134 | Pro | Et | Et | 〃 | 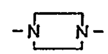 $-CH_2Ph-Cl$ (o) |

| Compound No. | $R_1$ | $R_2$ | $R_3$ | A | R |
|---|---|---|---|---|---|
| 141 | BU | $-(CH_2)_4-$ | | $-CH_2CH_2-$ | 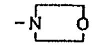 |
| 142 | BU | 〃 | | 〃 | 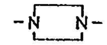 $-CH_2Ph$ |
| 143 | Bu | 〃 | | 〃 | 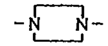 $-CH_2Ph-Cl$ (p) |
| 144 | Bu | 〃 | | 〃 | 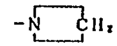 |
| 145 | Bu | 〃 | | 〃 | 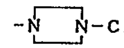 $-CH_2CH_2-OH$ |
| 146 | Bu | 〃 | | 〃 | 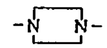 $-CH_2Ph-F$ (p) |
| 147 | $CH_2$ Ph | Me | Me | 〃 | -OCO-Me |
| 148 | Iso-Pro | Me | Me | 〃 | 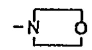 |
| 149 | 〃 | Me | Me | 〃 | 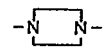 $-CH_2Ph$ |
| 150 | 〃 | Me | Me | 〃 | 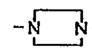 $-CH_2Ph-Cl$ (p) |
| 151 | 〃 | Me | Me | 〃 | 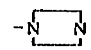 $-CH_2Ph-F$ (p) |
| 152 | sec-Bu | Me | Me | 〃 | 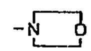 |
| 153 | 〃 | Me | Me | 〃 | 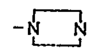 $-CH_2Ph$ |
| 154 | 〃 | Me | Me | 〃 | 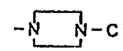 $-CH_2Ph-Cl$ (p) |
| 155 | 〃 | Me | Me | 〃 | 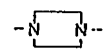 $-CH_2Ph-F$ (p) |
| 156 | Bu | Me | Me | 〃 | 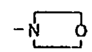 |

| Compound No. | $R_1$ | $R_2$ | $R_3$ | A | R |
|---|---|---|---|---|---|
| 157 | Bu | Me | Me | $-CH_2CH_2-$ | 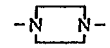 $-CH_2Ph$ |
| 158 | Bu | Me | Me | 〃 | 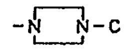 $-CH_2Ph-Cl$ (p) |
| 159 | Iso-Pro | Et | Et | 〃 | 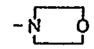 |
| 160 | 〃 | Et | Et | 〃 | 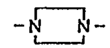 $-CH_2Ph$ |
| 161 | 〃 | Et | Et | 〃 | 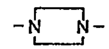 $-CH_2Ph-Cl$ (p) |
| 162 | 〃 | Et | Et | 〃 | 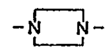 $-CH_2Ph-F$ (p) |
| 163 | 〃 | Et | Et | 〃 | 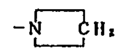 |
| 164 | 〃 | Et | Et | 〃 | 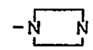 $-CH_2CH_2-OH$ |
| 165 | 〃 | Et | Et | 〃 | 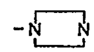 $-CH_2Ph-Cl$ (m) |
| 166 | Iso-Bu | Et | Et | 〃 | 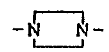 $-CH_2Ph-F$ (p) |
| 167 | 〃 | Et | Et | 〃 | 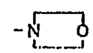 |
| 168 | 〃 | Et | Et | 〃 | 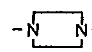 $-CH_2Ph-Cl$ (p) |
| 170 | 〃 | Et | Et | 〃 | 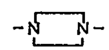 $-CH_2Ph$ |
| 171 | sec-Bu | Et | Et | 〃 | 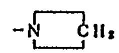 |
| 172 | 〃 | Et | Et | 〃 | 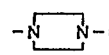 $-CH_2CH_2-OH$ |

| Compound No. | $R_1$ | $R_2$ | $R_3$ | A | R |
|---|---|---|---|---|---|
| 173 | sec−Bu | Et | Et | $-CH_2CH_2-$ | 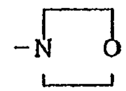 |
| 174 | 〃 | Et | Et | 〃 | 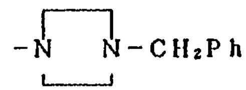 |
| 175 | 〃 | Et | Et | 〃 | 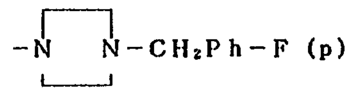 |
| 176 | 〃 | Et | Et | 〃 | 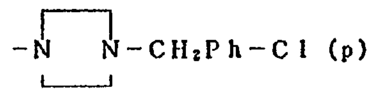 |
| 177 | 〃 | Et | Et | 〃 | 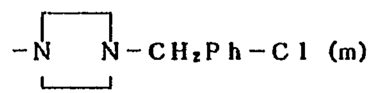 |
| 178 | | Et | Et | 〃 | 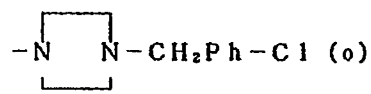 |
| 179 | Bu | Et | Et | 〃 | 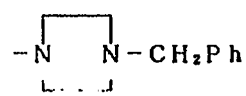 |

| | | | | | |
|---|---|---|---|---|---|
| 180 | Bu | Et | Et | 〃 | 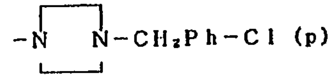 |
| 181 | Bu | Et | Et | 〃 | 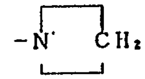 |
| 182 | Bu | Et | Et | 〃 | 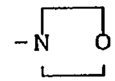 |
| 183 | Bu | Et | Et | 〃 | 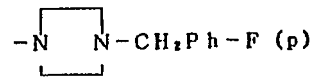 |
| 185 | Bu | Et | Et | 〃 | 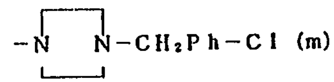 |
| 187 | Et | Et | Et | 〃 | 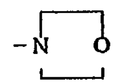 |
| 188 | Et | Et | Et | 〃 | 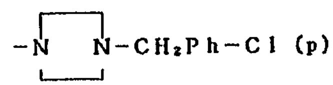 |
| 189 | Et | Et | Et | 〃 | 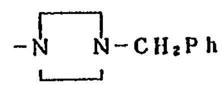 |

| 化合物番号 No. | $R_1$ | $R_2$ | $R_3$ | A | R |
|---|---|---|---|---|---|
| 190 | Et | Me | Me | $-CH_2CH_2-$ | 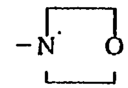 |
| 191 | Et | Me | Me | ″ | 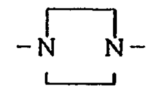 $-CH_2Ph-Cl$ (p) |
| 192 | Et | Me | Me | ″ | 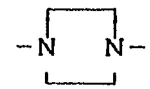 $-CH_2Ph-F$ (p) |
| 193 | Et | Me | Me | ″ | 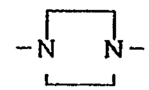 $-CH_2Ph-OMe$ (p) |
| 250 | $(CH_2)_4CH_3$ | $-(CH_2)_4-$ | | ″ | 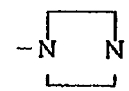 $-CH_2Ph-Cl$ (p) |
| 251 | ″ | ″ | | ″ | 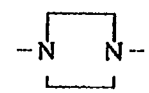 $-CH_2Ph-F$ (p) |
| 252 | ″ | ″ | | ″ | 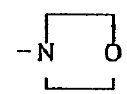 |

| | | | | |
|---|---|---|---|---|
| 253 | 〃 | 〃 | 〃 | 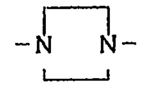$-Bu$ |
| 254 | 〃 | 〃 | 〃 | 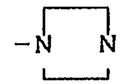$-CH_2Ph-OMe$ (p) |
| 255 | $CH_2)_5CH_3$ | 〃 | 〃 | 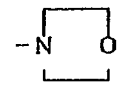 |
| 256 | 〃 | 〃 | 〃 | 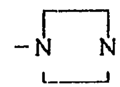$-CH_2Ph-Cl$ (p) |
| 257 | 〃 | 〃 | 〃 | 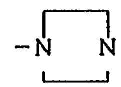$-CH_2Ph-NO_2$ (p) |
| 258 | 〃 | 〃 | 〃 | 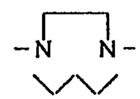$-CH_2Ph-F$ (p) |
| 259 | $CH_2)_6CH_3$ | 〃 | 〃 | 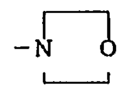 |
| 260 | 〃 | 〃 | 〃 | 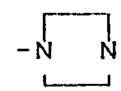$-CH_2Ph-Cl$ (p) |

| 化合物番号 No. | $R_1$ | $R_2$ $R_3$ | A | R |
|---|---|---|---|---|
| 261 | $(CH_2)_6CH_3$ | $-(CH_2)_4-$ | $-CH_2CH_2-$ | 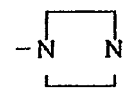 $-CH_2Ph-NO_2$ (p) |
| 262 | 〃 | 〃 | 〃 | 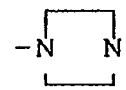 $-CH_2Ph-F$ (p) |
| 263 | 〃 | 〃 | 〃 | 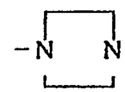 $-Bu$ |
| 264 | 〃 | 〃 | 〃 | 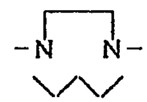 $-CH_2Ph-Cl$ (p) |
| 265 | $CH_2)_7CH_3$ | 〃 | 〃 | 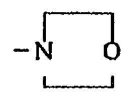 |
| 266 | 〃 | 〃 | 〃 | 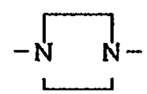 $-CH_2Ph-Cl$ (p) |
| 267 | 〃 | 〃 | 〃 | 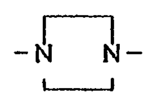 $-CH_2Ph-OMe$ (p) |

| | | | | |
|---|---|---|---|---|
| 268 | 〃 | 〃 | 〃 | 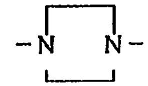$-CH_2Ph-NO_2$ (p) |
| 269 | 〃 | 〃 | 〃 | 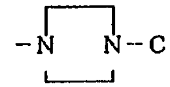$-CH_2Ph-F$ (p) |
| 270 | 〃 | 〃 | 〃 | 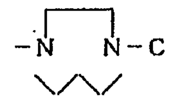$-CH_2Ph-F$ (p) |
| 271 | $CH_2)$ ▪$CH_3$ | 〃 | 〃 | 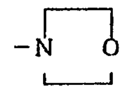 |
| 272 | 〃 | 〃 | 〃 | 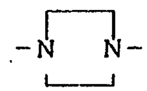$-CH_2Ph-Cl$ (p) |
| 273 | 〃 | 〃 | 〃 | 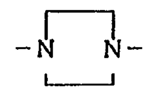$-CH_2Ph-F$ (p) |
| 274 | 〃 | 〃 | 〃 | 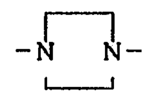$-CH_2Ph-NO_2$ (p) |
| 275 | 〃 | 〃 | 〃 | 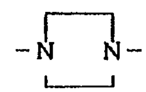$-CH_2Ph-OMe$ (p) |

| 化合物番号 N | $R_1$ | $R_2$ $R_3$ | A | R |
|---|---|---|---|---|
| 276 | $(CH_2)_{11}CH_3$ | $-(CH_2)_4-$ | $-CH_2CH_2-$ | 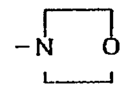 |
| 277 | 〃 | 〃 | 〃 | 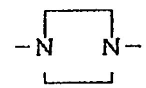 $-CH_2Ph-Cl$ (p) |
| 278 | 〃 | 〃 | 〃 | 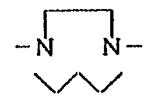 $-CH_2Ph-F$ (p) |
| 279 | 〃 | 〃 | 〃 | 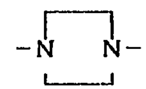 $-CH_2Ph-NO_2$ (p) |
| 280 | $(CH_2)_{13}CH_3$ | 〃 | 〃 | 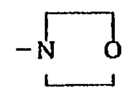 |
| 281 | 〃 | 〃 | 〃 | 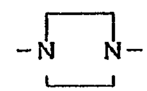 $-CH_2Ph-OMe$ (p) |
| 282 | 〃 | 〃 | 〃 | 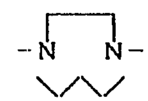 $-CH_2Ph-F$ (p) |

| | | | | |
|---|---|---|---|---|
| 283 | $(CH_2)_{15}CH_3$ | 〃 | 〃 | 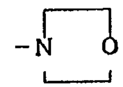 |
| 284 | 〃 | 〃 | 〃 | 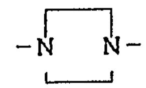$-CH_2Ph-Cl$ (p) |
| 285 | 〃 | 〃 | 〃 | 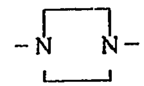$-CH_2Ph-NO_2$ (p) |
| 286 | 〃 | 〃 | 〃 | 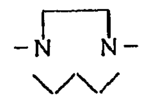$-CH_2Ph-Cl$ (p) |
| 287 | Et | Me Me | 〃 | 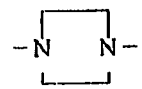$-CH_2Ph$ |
| 288 | Et | Me Me | 〃 | 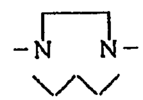$-CH_2Ph-Cl$ (p) |
| 289 | Et | Me Me | 〃 | 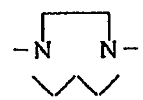$-CH_2Ph-F$ (p) |
| 290 | Et | Me Me | 〃 | 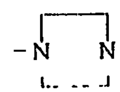$-CH_2Ph-NO_2$ (p) |

| 化合物番号 No. | $R_1$ | $R_2$ | $R_3$ | A | R |
|---|---|---|---|---|---|
| 291 | $CH_2Ph$ | Et | Et | $-CH_2CH_2-$ | 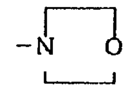 |
| 292 | 〃 | Et | Et | 〃 | 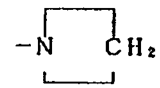 |
| 293 | 〃 | Et | Et | 〃 | 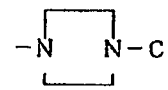$-CH_2Ph$ |
| 294 | 〃 | Et | Et | 〃 | 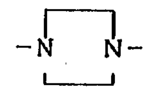$-CH_2Ph-Cl$ (p) |
| 295 | 〃 | Et | Et | 〃 | 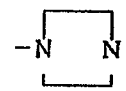$-CH_2Ph-Cl_2$ (2, 4) |
| 296 | 〃 | Et | Et | 〃 | 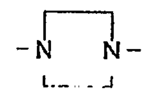$-CH_2Ph-F$ (p) |
| 297 | 〃 | Et | Et | 〃 | 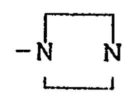$-CH_2Ph-OMe$ (p) |

| | | | | |
|---|---|---|---|---|
| 305 | CH₂)₅CH₃ | $-(CH_2)_4-$ | 〃 | 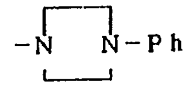 |
| 306 | 〃 | 〃 | 〃 | 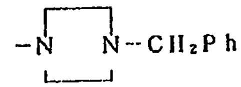 |
| 307 | 〃 | 〃 | 〃 | 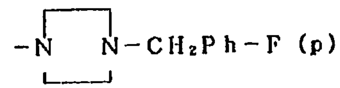 |
| 308 | 〃 | 〃 | 〃 | 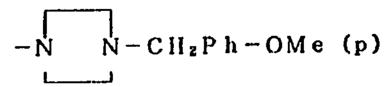 |
| 309 | 〃 | 〃 | 〃 | 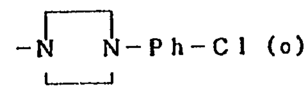 |
| 310 | 〃 | 〃 | 〃 | 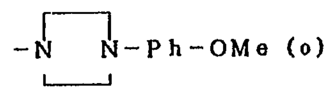 |
| 311 | 〃 | 〃 | 〃 | 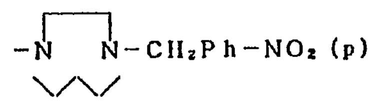 |
| 312 | 〃 | · | 〃 | 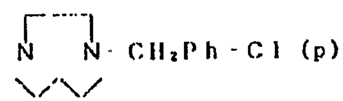 |

| 化合物番号 No. | $R_1$ | $R_2$ $R_3$ | A | R |
|---|---|---|---|---|
| 313 | $(CH_2)_5CH_3$ | $-(CH_2)_4-$ | $-CH_2CH_2-$ | 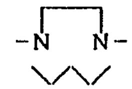 $-CH_2Ph-OMe$ (p) |
| 314 | $(CH_2)_6CH_3$ | 〃 | 〃 | 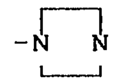 $-CH_2Ph$ |
| 315 | 〃 | 〃 | 〃 | 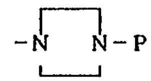 $-Ph$ |
| 316 | 〃 | 〃 | 〃 | 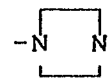 $-Ph-OMe$ (o) |
| 317 | 〃 | 〃 | 〃 | 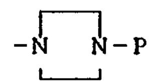 $-Ph-Cl$ (o) |
| 318 | 〃 | 〃 | 〃 | 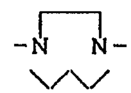 $-CH_2Ph-F$ (p) |
| 319 | 〃 | 〃 | 〃 | 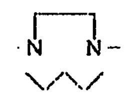 $-CH_2Ph-OMe$ (p) |
| 320 | 〃 | 〃 | 〃 | 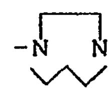 $-CH_2Ph-NO_2$ (p) |

| | | | | |
|---|---|---|---|---|
| 321 | CH$_2$)$_7$CH$_3$ | 〃 | 〃 | 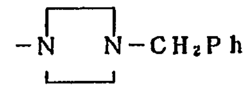 |
| 322 | 〃 | 〃 | 〃 | 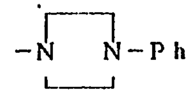 |
| 323 | 〃 | 〃 | 〃 | 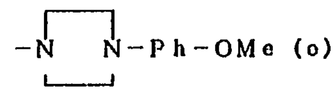 |
| 324 | 〃 | 〃 | 〃 | 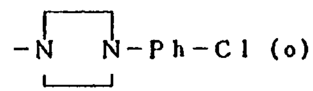 |
| 325 | 〃 | 〃 | 〃 | 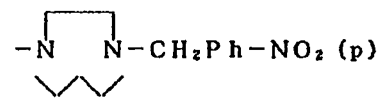 |
| 326 | 〃 | 〃 | 〃 | 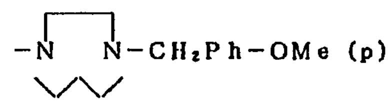 |
| 327 | 〃 | 〃 | 〃 | 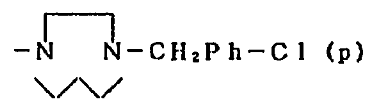 |

| 化合物番号 No. | $R_1$ | $R_2$ $R_3$ | A | R |
|---|---|---|---|---|
| 328 | $(CH_2)_8CH_3$ | $-(CH_2)_4-$ | $-CH_2CH_2-$ | 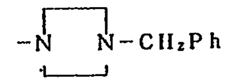 |
| 329 | 〃 | 〃 | 〃 | 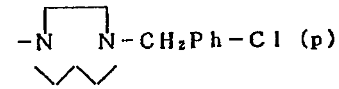 |
| 330 | 〃 | 〃 | 〃 | 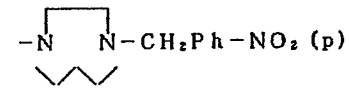 |
| 331 | 〃 | 〃 | 〃 | 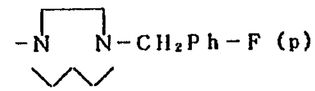 |
| 332 | 〃 | 〃 | 〃 | 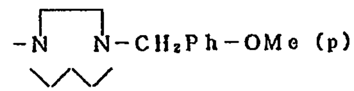 |
| 334 | $CH_2)_9CH_3$ | 〃 | 〃 | 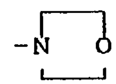 |
| 335 | 〃 | 〃 | 〃 | 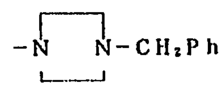 |
| 336 | 〃 | 〃 | 〃 | 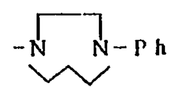 |

| | | | | | |
|---|---|---|---|---|---|
| 337 | ″ | | ″ | ″ | 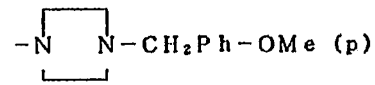 |
| 338 | ″ | | ″ | ″ | 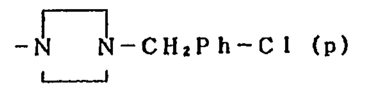 |
| 339 | ″ | | ″ | ″ | 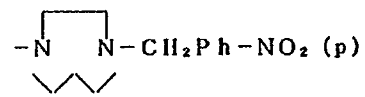 |
| 340 | ″ | | ″ | ″ | 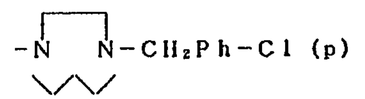 |
| 374 | $H_2Ph$ | Et | Et | ″ | 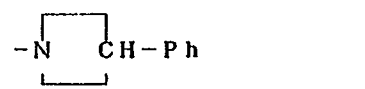 |
| 375 | ″ | Et | Et | ″ | 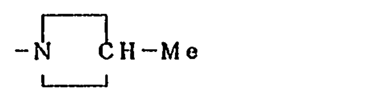 |
| 378 | ″ | Et | Et | ″ | 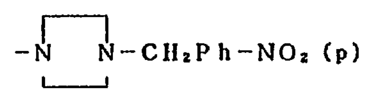 |

| Compound No. | $R_1$ | $R_2$ | $R_3$ | A | R |
|---|---|---|---|---|---|
| 380 | $CH_2Ph$ | Et | Et | $-CH_2CH_2-$ | 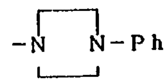 |
| 384 | $CH_2Ph-Cl$ (p) | Et | Et | 〃 | 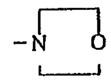 |
| 385 | 〃 | Et | Et | 〃 | 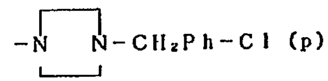 |
| 386 | 〃 | Et | Et | 〃 | 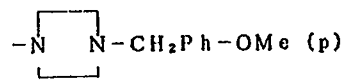 |
| 387 | 〃 | Et | Et | 〃 | 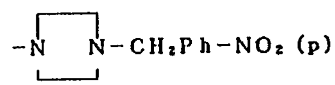 |
| 454 | Et | Me | Me | 〃 | 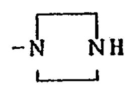 |
| 455 | Et | Me | Me | 〃 | 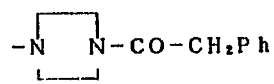 |
| 456 | Et | Me | Me | 〃 | 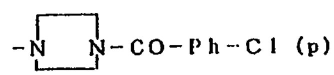 |

| | | | | | |
|---|---|---|---|---|---|
| 457 | Et | Me | Me | 〃 | − 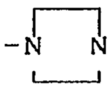 −CO−CH$_2$− (2−チエニル) |
| 458 | Et | Me | Me | 〃 | − 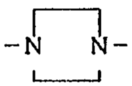 −CO−Ph−NO$_2$ (p) |
| 459 | Et | Me | Me | 〃 | − 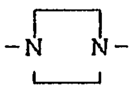 −CO−CH$_2$Ph−NO$_2$ (p) |
| 460 | Et | Me | Me | 〃 | − 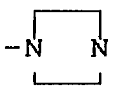 −CO−Ph−OMe (p) |
| 461 | Et | Me | Me | 〃 | − 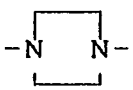 −CO−Ph−OMe$_2$ (2, 3) |
| 462 | Et | Me | Me | 〃 | − 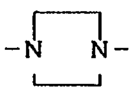 −CO−Ph−OMe$_2$ (2, 4) |
| 463 | Et | Me | Me | 〃 | − 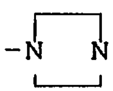 −CO−Ph−OMe$_2$ (3, 4) |

| 化合物番号 No. | $R_1$ | $R_2$ | $R_3$ | A | R |
|---|---|---|---|---|---|
| 464 | Et | Me | Me | $-CH_2CH_2-$ | 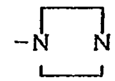 $-CO-Ph-OMe_3$ (3, 4, 5) |
| 465 | Et | Me | Me | 〃 | 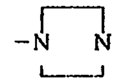 $-SO_2-Ph-Cl$ (p) |
| 467 | $CH_2Ph-Cl$ (p) | Et | Et | 〃 | 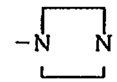 $-CH_2Ph$ |
| 470 | $CH_2Ph$ | $-(CH_2)_4-$ | | 〃 | 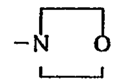 |
| 471 | 〃 | 〃 | | 〃 | 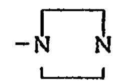 $-CH_2Ph$ |
| 472 | 〃 | 〃 | | 〃 | 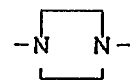 $-Ph$ |
| 473 | 〃 | 〃 | | 〃 | 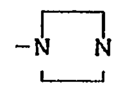 $-CH_2Ph-Cl$ (p) |
| 474 | 〃 | 〃 | | 〃 | 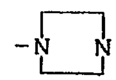 $-CH_2Ph-OMe$ (p) |

| | | | | |
|---|---|---|---|---|
| 475 | 〃 | 〃 | 〃 | 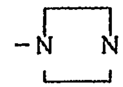$-CH_2Ph-F$ (p) |
| 476 | $CH_2CH_2Ph$ | 〃 | 〃 | 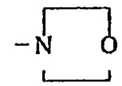 |
| 477 | 〃 | 〃 | 〃 | 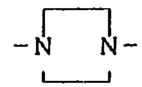$-CH_2Ph$ |
| 478 | 〃 | 〃 | 〃 | 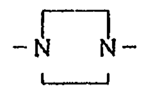$-Ph$ |
| 479 | 〃 | 〃 | 〃 | 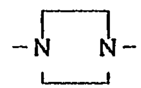$-CH_2Ph-Cl$ (p) |
| 480 | 〃 | 〃 | 〃 | 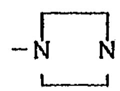$-CH_2Ph-OMe$ (p) |
| 481 | Et | $-(CH_2)_4-$ | $-CH_2CH_2-$ | 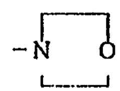 |

| 化合物番号 No. | $R_1$ | $R_2$ $R_3$ | A | R |
|---|---|---|---|---|
| 482 | Et | $-(CH_2)_4-$ | $-CH_2CH_2-$ | 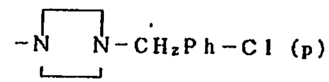 |
| 483 | Et | 〃 | 〃 | 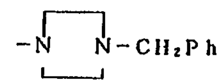 |
| 484 | Et | 〃 | 〃 | 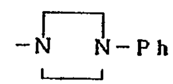 |
| 485 | Et | 〃 | 〃 | 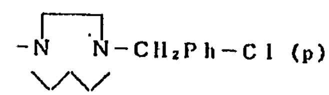 |
| 486 | Et | 〃 | 〃 | 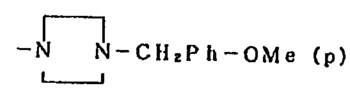 |
| 487 | $(CH_2)_{13}CH_3$ | 〃 | 〃 | 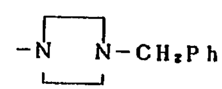 |
| 488 | 〃 | 〃 | 〃 | 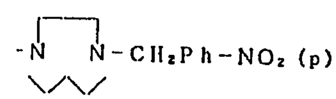 |
| 489 | 〃 | 〃 | 〃 | 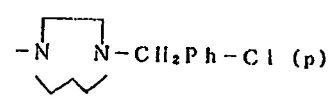 |

| | | | | | |
|---|---|---|---|---|---|
| 490 | 〃 | 〃 | | 〃 | 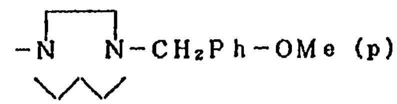 |
| 611 | Me | Me | Me | 〃 | 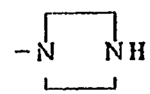 |
| 612 | Ph | Me | Me | 〃 | Cl |
| 613 | $H_2Ph$ | Me | Me | 〃 | Cl |
| 614 | Pro | $-(CH_2)_4-$ | | 〃 | -OH |
| 615 | Pro | 〃 | | 〃 | -S-Ph |
| 616 | Pro | Me | Me | 〃 | -OH |
| 617 | iso-Bu | Me | Me | 〃 | -OH |

| Compound No. | $R_1$ | $R_2$ | $R_3$ | A | R |
|---|---|---|---|---|---|
| 618 | iso-Bu | $-(CH_2)_4-$ | | $-CH_2CH_2-$ | $-OH$ |
| 619 | 〃 | Me | Me | 〃 | 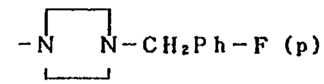 |
| 620 | Pro | Et | Et | 〃 | $-OH$ |
| 621 | Bu | $-(CH_2)_4-$ | | 〃 | $-OH$ |
| 622 | iso-Pro | Me | Me | 〃 | $-OH$ |
| 623 | sec-Bu | Me | Me | 〃 | $-OH$ |
| 624 | Bu | Me | Me | 〃 | $-OH$ |
| 625 | iso-Pro | Et | Et | 〃 | $-OH$ |
| 626 | iso Bu | Et | Et | 〃 | OH |
| 627 | sec Bu | Et | Et | 〃 | $-OH$ |

| | | | | |
|---|---|---|---|---|
| 628 | Bu | Et Et | 〃 | −OH |
| 629 | Et | Et Et | 〃 | −OH |
| 630 | Et | Me Me | 〃 | −OH |
| 631 | $(CH_2)_4CH_3$ | $-(CH_2)_4-$ | 〃 | −OH |
| 632 | $(CH_2)_5CH_3$ | 〃 | 〃 | −OH |
| 633 | $(CH_2)_6CH_3$ | 〃 | 〃 | −OH |
| 634 | $(CH_2)_7CH_3$ | 〃 | 〃 | −OH |
| 635 | $(CH_2)_8CH_3$ | 〃 | 〃 | −OH |
| 636 | $(CH_2)_9CH_3$ | 〃 | 〃 | −OH |

| Compound No. | $R_1$ | $R_2$ $R_3$ | A | R |
|---|---|---|---|---|
| 637 | $(CH_2)_{11}CH_3$ | $-(CH_2)_4-$ | $-CH_2CH_2$ | $-OH$ |
| 638 | $(CH_2)_{13}CH_3$ | 〃 | 〃 | $-OH$ |
| 639 | $(CH_2)_{15}CH_3$ | 〃 | 〃 | $-OH$ |
| 640 | $CH_2Ph$ | Et Et | 〃 | $-OH$ |
| 641 | $CH_2Ph-Cl$ (p) | Et Et | 〃 | $-OH$ |
| 642 | Me | Me Me | 〃 | 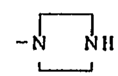 |
| 643 | $CH_2Ph$ | $-(CH_2)_4-$ | 〃 | $-OH$ |
| 644 | $CH_2Ph$ | 〃 | 〃 | Cl |
| 645 | $CH_2CH_2Ph$ | 〃 | 〃 | $-OH$ |
| 646 | Et | 〃 | 〃 | $-OH$ |
| 647 | $CH_2Ph-Cl$ (p) | Et Et | 〃 | Cl |

[Effect of the invention]

Pharmacological actions of the present compound [1] are illustrated hereinbelow. All the compounds [1] used in the pharmacological tests are applied in the form of hydrochloride.

(1) Platelet aggregation inhibition:

Sample solution containing a compound [1] (final concentration 500 or 100 μM) is added in rabbit platelet plasma added with 1/10 volume of 3.8% sodium citrate and incubated at 37°C for 3 minutes. A platelet activation factor (PAF, final concentration 10-50 ng/ml) or collagen (final concentration 2.5 μg/ml) as an aggregating agent are added thereto, and platelet aggregation activity is measured by aggrigometer. Result of assays on PAF induced aggregation is shown in Table 2, and that of collagen induced aggregation is shown in Table 3, in which showing clear platelet aggregation inhibitory activities of the compound [1] of the present invention.

Table 2. Platelet aggregation inhibitory action on PAF induced aggregation

| Compound No. | Concentration μM | Inhibition % | Compound No. | Concentration μM | Inhibition % |
|---|---|---|---|---|---|
| 114 | 500 | 63 | 174 | 500 | 96 |

| | | | | | |
|---|---|---|---|---|---|
| 115 | 〃 | 88 | 175 | 〃 | 94 |
| 116 | 〃 | 76 | 176 | 〃 | 81 |
| 120 | 〃 | 83 | 177 | 〃 | 79 |
| 126 | 〃 | 54 | 178 | 〃 | 94 |
| 128 | 〃 | 83 | 179 | 〃 | 93 |
| 129 | 〃 | 79 | 180 | 〃 | 90 |
| 132 | 〃 | 72 | 183 | 〃 | 85 |
| 133 | 〃 | 63 | 185 | 〃 | 61 |
| 134 | 〃 | 57 | 188 | 〃 | 86 |
| 142 | 〃 | 82 | 189 | 〃 | 69 |
| 143 | 〃 | 87 | 191 | 〃 | 62 |
| 145 | 〃 | 52 | 253 | 100 | 93 |
| 146 | 〃 | 87 | 263 | 〃 | 70 |
| 154 | 〃 | 54 | 306 | 〃 | 60 |
| 158 | 〃 | 64 | 308 | 〃 | 40 |
| 160 | 〃 | 97 | 310 | 〃 | 46 |
| 161 | 〃 | 93 | 313 | 〃 | 48 |
| 162 | 〃 | 93 | 316 | 〃 | 49 |
| 163 | 〃 | 62 | 321 | 〃 | 43 |
| 165 | 〃 | 92 | 323 | 〃 | 43 |
| 166 | 〃 | 87 | 326 | 〃 | 51 |
| 168 | 〃 | 76 | 332 | 〃 | 46 |
| 170 | 〃 | 80 | 386 | 〃 | 71 |
| 172 | 〃 | 57 | 387 | 〃 | 41 |
| 173 | 〃 | 61 | | | |

Table 3 Platelet aggregation inhibitory action on collagen induced aggregation

| Compound No. | Concentration μM | Inhibition % | Compound No. | Concentration μM | Inhibition % |
|---|---|---|---|---|---|
| 114 | 100 | 50 | 289 | 100 | 58 |
| 115 | 〃 | 50 | 292 | 〃 | 50 |
| 251 | 〃 | 78 | 384 | 〃 | 76 |
| 252 | 〃 | 42 | 386 | 〃 | 71 |
| 253 | 〃 | 52 | 467 | 〃 | 80 |
| 255 | 〃 | 48 | 471 | 〃 | 81 |
| 263 | 〃 | 73 | 473 | 〃 | 64 |
| 265 | 〃 | 45 | 474 | 〃 | 81 |
| 287 | 〃 | 50 | 475 | 〃 | 81 |
| 288 | 〃 | 72 | 477 | 〃 | 72 |

(2) Vasodilation activity:

Dog, pretreated with morphine 81.5 mg/kg, sc), is anesthesized with urethane 8450 mg/kg, iv) and α-chloralos (45 mg/kg, iv), and fixed at dosal position. Right femoral arterial blood is introduced into a left femoral artery via perfusion pump, and Sterling's resistance is connected in the exosomatic circulation system to perfuse in a left back limb at constant pressure. Perfusion pressure is set on a valve slightly higher than that of an average blood pressure of the animal. Sample (100 μg) dissolved in physiological saline is administered into a right femoral artery, and changes of blood flow is measured. Vasodilation activity is determined as a specific activity by setting the increased ratio of blood flow as 100% when papaverin 30 μg is administered intra arterially. Result is shown in Table 4 in which the compound [1] of the present invention has clear vasodilation activity.

Table 4 Vasodilation activity

| Compound No. | Vasodilation activity % | Compound No. | Vasodilation activity % |
|---|---|---|---|
| 023 | 118 | 158 | 139 |
| 024 | 182 | 160 | 117 |
| 025 | 112 | 161 | 214 |
| 114 | 174 | 162 | 212 |
| 115 | 210 | 165 | 222 |
| 116 | 147 | 166 | 153 |
| 119 | 132 | 168 | 205 |
| 120 | 223 | 170 | 178 |
| 122 | 161 | 171 | 122 |
| 124 | 278 | 174 | 179 |
| 125 | 220 | 175 | 151 |
| 126 | 310 | 176 | 159 |
| 128 | 155 | 177 | 122 |

| | | | |
|---|---|---|---|
| 129 | 201 | 178 | 111 |
| 131 | 158 | 179 | 139 |
| 132 | 231 | 180 | 136 |
| 133 | 251 | 181 | 125 |
| 134 | 143 | 183 | 107 |
| 142 | 151 | 185 | 115 |
| 143 | 119 | 188 | 188 |
| 144 | 120 | 189 | 133 |
| 146 | 125 | 191 | 165 |
| 149 | 122 | 193 | 226 |
| 150 | 229 | 254 | 232 |
| 151 | 121 | 287 | 170 |
| 153 | 197 | 288 | 232 |
| 157 | 162 | 297 | 229 |

(3) Antioxidant activity:

Antioxidant activity is determined according to a method of Stocks et al. [Clin. Sci. Mol. Med., 47: 215 (1974)]. Rat cerebrum is added in ice-cooled 40 mM phosphate saline buffer solution (PBS) (pH 7.4, buffer 4 ml per cerebrum 1 g), homogenized and centrifuged (1000 X g, 4 °C, 10 min.) to obtain supernatant solution. The supernatant solution is diluted for 5 times with the above ice-cooled PBS solution, and to a 0.9 ml thereof is added the sample of compound [1] (0.1 ml, final concentration 100 μM) dissolved in ethanol, incubated at 37 °C for 15 minutes, added 35% perchloric acid (0.2 ml), ice-cooled to stop the reaction and centrifuged (1300 X g, 4 °C, 10 min.). Thiobarbituric acid (5 g/lit. of 50% acetic acid, 0.5 ml) is added to the supernatant solution (1 ml), heatd at 100°C for 30 minutes and ice-cooled to measure absorbancy at 532 nm. The thus formed amount of lipoperoxide is expressed as an amount of malondialdehyde. Result is shown in Table 5 in which a compound [1] of the present invention has an inhibitory action for lipoperoxide generation.

Table 5 Antioxidant activity

| Compound No. | Inhibition % | Compound No. | Inhibition % |
|---|---|---|---|
| 115 | 44 | 307 | 50 |
| 119 | 41 | 308 | 57 |
| 129 | 53 | 309 | 71 |
| 132 | 53 | 310 | 79 |
| 133 | 51 | 311 | 89 |
| 134 | 49 | 312 | 93 |
| 142 | 48 | 313 | 89 |
| 143 | 53 | 314 | 75 |
| 150 | 45 | 315 | 82 |
| 154 | 51 | 316 | 86 |
| 158 | 44 | 317 | 68 |
| 160 | 41 | 318 | 82 |
| 161 | 76 | 319 | 89 |

| | | | |
|---|---|---|---|
| 162 | 47 | 320 | 86 |
| 165 | 70 | 321 | 75 |
| 168 | 59 | 322 | 79 |
| 175 | 49 | 323 | 75 |
| 176 | 88 | 324 | 64 |
| 177 | 73 | 325 | 92 |
| 178 | 67 | 326 | 88 |
| 180 | 69 | 327 | 92 |
| 183 | 41 | 328 | 72 |
| 185 | 63 | 329 | 88 |
| 256 | 66 | 330 | 92 |
| 258 | 87 | 331 | 88 |
| 260 | 62 | 332 | 80 |
| 264 | 79 | 334 | 56 |
| 267 | 68 | 335 | 80 |
| 268 | 77 | 336 | 96 |
| 269 | 70 | 337 | 88 |
| 270 | 93 | 338 | 80 |
| 272 | 83 | 339 | 72 |
| 273 | 77 | 340 | 92 |
| 274 | 77 | 378 | 52 |
| 275 | 80 | 385 | 67 |
| 276 | 67 | 386 | 50 |
| 277 | 70 | 387 | 77 |
| 278 | 87 | 467 | 61 |
| 279 | 73 | 472 | 53 |

| | | | |
|---|---|---|---|
| 280 | 67 | 473 | 58 |
| 281 | 77 | 478 | 59 |
| 282 | 87 | 479 | 53 |
| 283 | 53 | 487 | 72 |
| 295 | 55 | 488 | 84 |
| 305 | 61 | 489 | 78 |
| 306 | 54 | 490 | 75 |

As explained hereinabove, a compound [1] of the present invention or its salt has an inhibitory action for platelet aggregation, vasodilating activity and/or inhibitory action lipoperoxide generation, and is useful as pharmaceuticals for improvement on circulation and metabolic disorder.

[Examples]

Following examples illustrated the present invention but are not construed as limiting.

In the examples, Rf value of silica-gel thin layer chromatography (TLC) is a value measured, if not specified, by using the following carrier and developing solvent.
Carrier: silica-gel, Kieselgel 60 $F_{254}$ (Merck)
Developper: chloroform - methanol (20 : 1)

Physical properties of the compound [1] obtained in the following examples are illustrated in Table 27.

Example 1

1-(2-hydroxyethyl)-3,5,6-trimethyl-2-oxo-1,2- dihydropyrazine (compound 026):

2-hydroxy-3,5,6-trimethylpyrazine (13.8 g, 0.1 M) and 5N-NaOH (100 ml, 0.5 M) were added to t-butanol (200 ml). Ethylene chlorohydrin (40.3 g, 0.5 M) was added therein and stirred at 60 °C for 2 hours. Water was added to the reaction mixture, extracted 10 times with chloroform (100 ml), dried the extract with anhydrous magnesium sulfate and concentrated in vacuo. Residue was charged on a column of silica-gel (Wako Pure Chem. Co., C-200) and eluted with chloroform-methanol (300 : 1) to obtain the product. (14.4 g, Yield: 79.1%) dihydrochloride: m.p.: 140 - 144°C.

Examples 2-9

1-(2-substituted ethyl)-3,5,6-trimethyl-2-oxo-1,2-dihydropyrazine:

Thionylchloride (0.86 ml, 12mM) in chloroform (0.5 ml) was added dropwise under ice-cooling to the compound 026 (1.82 g, 10 mM) suspended in chloroform (5 ml) and stirred at room temperature for 3 hours to chlorinate. Reaction mixture was poured into diluted aqueous $Na_2CO_3$, extracted 3 times with chloroform, dried the extract with anhydrous sodium sulfate, and concentrated in vacuo.

Base (20mM) and triethylamine (2.8 ml, 20 mM) were added to the obtained chlorinated compound dissolved in benzene (60 ml) and refluxed. Reaction mixture was washed with diluted aqueous $Na_2CO_3$ and an aqueous layer was extracted 2 times with benzene. Combined benzene layer, which was dried with anhydrous sodium sulfate, was concentrated in vacuo. Residue was chromatographed on a silica-gel (80 g) column using elution solvent of chloroform-methanol to obtain the compounds in Table 6.

Kind of base, reflux time and ratio of chloroform-methanol mixture are shown in Table 6.

Examples 10-11

1-(2-substituted thioethyl)-3,5,6-trimethyl-2-oxo-1,2-dihydropyrazine:

A compound 026 (0.91 g, 5 mM) was chlorinated with thionylchloride according to the same method in Examples 2-9. The thus obtained chlorinated compound was dissolved in dimethylformamide (DMF, 10 ml), added sodium mercaptane (5 mM) and stirred at room temperature for 2 days. DMF was removed off in vacuo. Diluted aqueous $K_2CO_3$ solution was added to the residue, extracted with chloroform, dried by adding anhydrous sodium sulfate and concentrated in vacuo. Residue was chromatographed on a silica-gel (C-200, 50 g) column eluted with benzeneethyl acetate (5 : 1) to obtain the product in Table 7.

A kind of mercaptane is illustrated in Table 7.

Example 12

1,4-bis (2-(3,5,6-trimethyl-2-oxo-1,2-dihydropyrazine-1-yl)ethyl)-piperazine (compound 101):

Chloroform (3 ml) solution of thionylchloride (4.3 ml, 60 mM) was added dropwise under ice-cooling to a compound 026 (9.10 g, 50 mM) suspended in chloroform (15 ml). After stirring at room temperature for 3 hours, reaction mixture was poured into diluted aqueous $K_2CO_3$ and extracted with chloroform (200 ml). Chloroform layer was dried with anhydrous magnesium sulfate and concentrated in vacuo. Residue was dissolved in benzene (300 ml), added piperazine (2.15 g) and triethylamine (0.3 ml, 50 mM) and refluxed for 3 hours. Reaction mixture was washed with diluted aqueous $K_2CO_3$ and extracted the aqueous layer with chloroform. Chloroform layer was dried with anhydrous magnesium sulfate and concentrated in vacuo. Residue was charged on a column of silica-gel (c–200, 180 g), and eluted with chloroform-methanol (100:1) to obtain the compound 101. (7.60 g, Yield: 73,2%)

Example 13

1-(2-pyperazinoethyl)-3,5,6-trimethyl-2-oxo-1,2-dihydro pyrazine (compound 642):

In example 12, piperazine (17.2 g, 0.2 M) and triethylamine (28 ml, 0.2 M) were used and the other conditions were repeated according to the example 12 to obtain the compound 642 (5.52 g, Yield: 44.2%) which showed lower Rf value as compared with the compound 101.

Example 14

1-(2-hydroxyethyl)-3-phenyl-5,6-dimethyl-2-oxo-1,2-dihydropyrazine (compound 109):

Ethylene chlorohydrin (20.2 g, 0.25 M) was added to 2-hydroxy-3-phenyl-5,6-dimethyl-pyrazine (10.0 g, 50 mM) and 5N-NaOH (50 ml) dissolved in t-butanol (150 ml) and stirred at 60°C for 2.5 hours. Water was added to the reaction mixture, extracted with chloroform, dried with anhydrous sodium sulfate and concentrated in vacuo. Residue was charged on a column of silica-gel (C-200, 130 g) packed with chloroform and eluted with chloroform-methanol (100 : 1) to obtain the compound 109. (8.73 g, Yield: 71.5%)

Example 15

1-(2-chloroethyl)-3-phenyl-5,6-dimethyl-2-oxo-1,2-dihydropyrazine (compound 612):

Thionylchloride (0.42 ml, 6.0 mM) was added dropwise under ice-cooling to a compound 109 (1.22 g, 50 mM) dissolved in chloroform (10 ml) and stirred at room temperature for 3 hours. Chloroform was added to the reaction mixture, washed with diluted aqueous $K_2CO_3$ and further extracted the aqueous layer with chloroform. Chloroform layer was combined, dried with anhydrous magnesium sulfate and concentrated in vacuo. Residue was charged on a column of silica-gel (C-200, 25 g), and eluted with chloroform to obtain the compound 612. (0.59 g, Yield: 42.7%)

Examples 16-17

1-(2-substituted ethyl)-3-phenyl-5,6-dimethyl-2-oxo- 1,2-dihydropyrazine:

Base (5 mM) and triethylamine (0.70 ml, 5 mM) were added to a compound 612 (0.66 g, 2.5 mM) dissolved in benzene (15 ml) and refluxed. Reaction mixture was washed with diluted aqueous $K_2CO_3$, further extracted the aqueous layer with benzene. Benzene layer was combined, dried with anhydrous sodium sulfate and concentrated in vacuo. Residue was charged on a column of silica-gel (C-200, 65 g) and eluted with chloroform-methanol to obtain the compounds in Table 8.

A kind of base, reflux time and ratio of chloroform-methanol mixture are shown in Table 8.

Example 18

1-(2-hydroxyethyl)-3-benzyl-5,6-dimethyl-2-oxo-1,2-dihydropyrazine (compound 112):

Ethylene chlorohydrin (4.03 g, 50 mM) was added to 2-hydroxy-3-benzyl-5,6-dimethyl-pyrazine (2.14

g, 10 mM) and 5N-NaOH (10 ml) dissolved in t-butanol (30 ml), and stirred at 60°C for 2.5 hours. Water (100 ml) was added to the reaction mixture, extracted 3 times with chloroform, dried the extract with anhydrous magnesium sulfate and concentrated in vacuo. Residue was charged on a column of silica-gel (C-200, 25 g) and eluted with chloroform-methanol (100 : 1) to obtain the compound 122. (2.22 g, Yield: 86.0%)

Example 19

1-(2-chloroethyl)-3-benzyl-5,6-dimethyl-2-oxo-1,2-dihydropyrazine (compound 613):

Thionylchloride (0.42 ml, 6.0 mM) was added dropwise to a compound 112 (1.29 g, 5 mM) dissolved in chloroform (6 ml) under ice-cooling and stirred at room temperature for 5 hours. Chloroform (70 ml) was added to the reaction mixture, washed with diluted aqueous $K_2CO_3$ and further extracted twice the aqueous layer with chloroform. Chloroform layer was combined, dried with anhydrous magnesium sulfate and concentrated in vacuo. Residue was charged on a column of silica-gel (C-200, 25 g), packed with chloroform, and eluted with chloroform to obtain the compound 613. (1.00 g, Yield: 87.5%)

Examples 20-23

1-(2-substituted ethyl)-3-benzyl-5,6-dimethyl-2-oxo-1,2-dihydropyrazine :

Base (6.0 mM) and triethylamine (0.84 ml, 6.0 mM) were added to a compound 613 (0.83 g, 3.0 mM) dissolved in benzene (15 ml) and refluxed. Reaction mixture was washed with diluted aqueous $K_2CO_3$, further extracted the aqueous layer with benzene. Benzene layer was combined, dried with anhydrous sodium sulfate and concentrated in vacuo. Residue was charged on a column of silica-gel (C-200, 65 g) packed with chloroform and eluted with chloroform-methanol to obtain the compounds in Table 9.

A kind of base, reflux time and ratio of chloroform-methanol mixture are shown in Table 9.

Examples 24-28

1-(2-hydroxyethyl)-3-alkyl-5,6-dimethyl-2-oxo-1,2-dihydropyrazine :

Aqueous 5N-NaOH (10 ml, 50 mM), t-butanol (30 ml) and ethylene chlorohydrin (50 mM) were added to 2-hydroxy-3-alkyl-5,6-dimethyl-pyrazine (10 mM) and stirred at 60 °C. Water was added to the reaction mixture, extracted 3 times with chloroform, dried with anhydrous magnesium sulfate and concentrated in vacuo. Residue was charged on a column of silica-gel (C-200, 120 g) and eluted with chloroform-methanol to obtain the compounds (hydroxy ethyl form) in Table 10.

A kind of 2-hydroxy-3-alkyl-5,6-dimethyl-pyrazine indicated as a group $R_1$, its amount used, reaction time in hour, and ratio of mixture of chloroform-methanol are illustrated in Table 10.

Examples 29-46

1-(2-substituted ethyl)-3-alkyl-5,6-trimethyl-2-oxo-1,2-dihydropyrazine:

Thionylchloride (0.34 ml) was added dropwise under ice-cooling to the hydroxy ethyl form of compound (4 mM), obtained in Examples 24-28, dissolved in chloroform (5 ml) and stirred at room temperature for 1 hour. Chloroform was added to the reaction mixture, washed with diluted aqueous $K_2CO_3$, further extracted twice the aqueous layer with chloroform. Chloroform layer was combined, dried with anhydrous magnesium sulfate and concentrated in vacuo. Benzene (30ml), triethylamine (1.12 ml) and base (8 mM) were added to the residue and refluxed. Chloroform (100-120 ml) was added to the reaction mixture and washed with diluted aqueous $K_2CO_3$. Aqueous layer was extracted twice with chloroform. Chloroform layer was combined, dried with anhydrous magnesium sulfate and concentrated in vacuo. Residue was charged on a column of silica-gel (C-200, 60 g) and eluted with chloroform-methanol to obtain the compounds shown in Table 11.

Kind of starting material (hydroxy ethyl form), base, reflux time and ratio of chloroform-methanol mixture are shown in Table 11.

Examples 47-49

1-(2-hydroxyethyl)-3-alkyl-5,6-dimethyl-2-oxo-1,2,5,6,7,8-hexahydroquinoxaline:

Aqueous 5N-NaOH (10 ml, 50 mM), t-butanol (30 ml) and ethylene chlorohydrin (50 mM) were added to 2-hydroxy-3-alkyl-5,6,7,8-tetrahydroquinoxaline (10 mM) and stirred at 60 °C. Water was added to the reaction mixture, extracted with chloroform, dried with anhydrous magnesium sulfate and concentrated in vacuo. Residue was charged on a column of silica-gel (C-200) and eluted with chloroform-methanol to obtain the compounds (hydroxy ethyl form) in Table 12.

A kind of 2-hydroxy-3-alkyl-5,6,7,8-tetrahydroquinoxaline indicated as a group $R_1$, its amount used, reaction time in hour, and ratio of mixture of chloroform-methanol are illustrated in Table 12.

Examples 50-62

1-(2-substituted ethyl)-3-alkyl-2-oxo-1,2,5,6,7,8-hexahydroquinoxaline:

Thionylchloride (0.34 ml) was added dropwise under ice-cooling to the hydroxy ethyl form of compound (4 mM), obtained in Examples 47-49, dissolved in chloroform and stirred at room temperature for 1 hour. Chloroform was added to the reaction mixture, washed with diluted aqueous $K_2CO_3$, further extracted the aqueous layer with chloroform. Chloroform layer was combined, dried with anhydrous magnesium sulfate and concentrated in vacuo. Benzene (30ml), triethylamine (1.12 ml, 8mM) and base (8 mM) were added to the reactionmixture and refluxed. Chloroform (100 ml) was added to the reaction mixture, washed with diluted aqueous $K_2CO_3$, then extracted the aqeous layer with chloroform. Chloroform layer was combined, dried with anhydrous magnesium sulfate and concentrated in vacuo. Residue was charged on a column of silica-gel (C-200, 70 g) and eluted with chloroform-methanol to obtain the compounds shown in Table 13.

Kind of starting material (hydroxy ethyl form), base, reflux time and ratio of chloroform-methanol mixture are shown in Table 13.

Examples 63-68

1-(2-hydroxyethyl)-3-alkyl-5,6-diethyl-2-oxo-1,2-dihydropyrazine :

Aqueous 5N-NaOH (10 ml, 50 mM), t-butanol (30 ml) and ethylene chlorohydrin (50 mM) were added to 2-hydroxy-3-alkyl-5,6-diethyl-pyrazine (10 mM) and stirred at 60°C. Water was added to the reaction mixture, extracted with chloroform, dried with anhydrous sodium sulfate and concentrated in vacuo.

Residue was charged on a column silica-gel (C-200) packed with chloroform and eluted with chloroform-methanol to obtain the compounds in Table 14.

A kind of 2-hydroxy-3-alkyl-5,6-diethyl-pyrazine indicated as a group $R_1$, its amount used, reaction time in hour, an amount of silica-gel and ratio of mixture of chloroform-methanol are illustrated in Table 14.

Examples 69-101

1-(2-substituted ethyl)-3-alkyl-5,6-diethyl-2-oxo-1,2-dihydropyrazine:

Thionylchloride (0.34 ml) was added dropwise under ice-cooling to the hydroxy ethyl form of compound (4 mM), obtained in Examples 63-68, dissolved in chloroform (5 ml) and stirred at room temperature for 1 hour. Chloroform was added to the reaction mixture, washed with diluted aqueous $K_2CO_3$, further extracted twice the aqueous layer with chloroform. Chloroform layer was combined, dried with anhydrous magnesium sulfate and concentrated in vacuo. Benzene (30ml), triethylamine (1.12 ml, 8 mM) and base (8 mM) were added to the residue and refluxed. Chloroform (100 ml) was added to the reaction mixture and washed with diluted aqueous $K_2CO_3$. Further aqueous layer was extracted with chloroform. Chloroform layer was combined, dried with anhydrous magnesium sulfate and concentrated in vacuo. Residue was charged on a column of silica-gel (C-200, 60 g) packed with chloroform, and eluted with chloroform-methanol to obtain the compounds shown in Table 15.

A kind of starting material (hydroxy ethyl form), base, reflux time and ratio of chloroform-methanol mixture are shown in Table 15.

Example 102

1-(2-hydroxyethyl)-3-ethyl-5,6-dimethyl-2-oxo-1,2-dihydropyrazine (compound 630):

Ethylene chlorohydrin (32.21 g, 0.4 M) was added to 2-hydroxy-3-ethyl-5,6-dimethyl-pyrazine (12.16 g, 80 mM) and 5N-NaOH (80 ml) dissolved in t-butanol (240 ml), and stirred at 60 °C for 3 hours. Diluted aqueous $K_2CO_3$ was added to the reaction mixture, extracted with chloroform, and further extracted twice the aqueous layer with chloroform. Combined chloroform layer was dried with anhydrous magnesium sulfate and concentrated in vacuo. Residue was charged on a column of silica-gel (C-200, 250 g) packed with chloroform and eluted with chloroform-methanol (from 200 : 1 to 50 : 1) to obtain the compound 630. (12.31 g, Yield: 78.5%)

Examples 103-110

1-(2-substituted ethyl)-3-ethyl-5,6-dimethyl-2-oxo-1,2-dihydropyrazine:

Thionylchloride (0.43 ml) was added dropwise under ice-cooling to the compound (0.98 g, 5 mM) dissolved in chloroform (5 ml) and stirred at room temperature for 1 hour. Chloroform (50 ml) was added to the reaction mixture, washed with diluted aqueous $K_2CO_3$, further extracted twice the aqueous layer with chloroform (50 ml). Chloroform layer was combined, dried with anhydrous magnesium sulfate and concentrated in vacuo. Benzene (30ml), triethylamine (1.40 ml) and base (10 mM) were added to the residue and refluxed. Chloroform (100 ml) was added to the reaction mixture and washed with diluted aqueous $K_2CO_3$. Further aqueous layer was extracted twice with chloroform (50 ml). Chloroform layer was combined, dried with anhydrous magnesium sulfate and concentrated in vacuo. Residue was charged on a column of silica-gel (C-200, 70 g) packed with chloroform, and eluted with chloroform-methanol to obtain the compounds shown in Table 16.

A kind of base, reflux time and ratio of chloroform-methanol mixture are shown in Table 16.

Example 111

1-(2-hydroxyethyl)-3-benzyl-5,6-diethyl-2-oxo-1,2-dihydropyrazine (compound 640):

Ethylene chlorohydrin (20.15 g, 0.25 M) was added to 2-hydroxy-3-benzyl-5,6-diethyl-pyrazine (12.10 g, 50 mM) and 5N-NaOH (50 ml) dissolved in t-butanol (150 ml), and stirred at 60°C for 3 hours. A t-butanol kwas distilled off in vacuo, added water thereto and extracted 3 times with chloroform. Extract was dried with anhydrous magnesium sulfate and concentrated in vacuo. Residue was charged on a column of silica-gel (C-200, 230 g) packed with chloroform and eluted with chloroform-methanol (100 : 1) to obtain the compound 640. (12.31 g, Yield: 58.7%)

Examples 112-122

1-(2-substituted ethyl)-3-benzyl-5,6-dimethyl-2-oxo-1,2-dihydropyrazine:

Thionylchloride (0.34 ml) was added dropwise under ice-cooling to the compound 640 (1.15 g, 4.0 mM) dissolved in chloroform (5 ml) and stirred at room temperature for 1 hour. Chloroform (100 ml) was added to the reaction mixture, washed with diluted aqueous $K_2CO_3$, further extracted twice the aqueous layer with chloroform (30 ml). Chloroform layer was combined, dried with anhydrous sodium sulfate and concentrated in vacuo. Triethylamine (1.12 ml, 8 mM) and base (8 mM) were added to the residue dissolved in benzene (30 ml) and refluxed. The reaction mixture was washed with diluted aqueous $K_2CO_3$. Aqueous layer was extracted twice with benzene. Benzen layer was combined, dried with anhydrous sodium sulfate and concentrated in vacuo. Residue was charged on a column of silica-gel (C-200, 60 g) and eluted with chloroform-methanol to obtain the compounds shown in Table 17.

Kind of base, reflux time and a ratio of chloroform-methanol mixture are shown in Table 17.

Example 123

1-(2-hydroxyethyl)-3-p-chlorobenzyl-5,6-dimethyl-2-oxo-1,2-dihydropyrazine (compound 641):

Ethylene chlorohydrin (16.11 g, 0.20 M) was added to 2-hydroxy-3-p-chlorobenzyl-5,6-dimethyl-pyrazine (11.06 g, 40 mM) dissolved in aqueous 5N-NaOH (40 ml) and t-butanol (120 ml), and stirred at 60°C for 3 hours. A t-butanol was distil led off in vacuo. Diluted aqueous $K_2CO_3$ was added to the residue, extracted three times with chloroform, dried with anhydrous sodium sulfate and concentrated in vacuo. Residue was charged on a column of silica-gel (C-200, 300 g) packed with chloroform and eluted with chloroform-methanol (100 : 1) to obtain the compound 641. (9.73 g, Yield: 75.9%)

Example 124

1-(2-chloroethyl)-3-p-chlorobenzyl-5,6-diethyl-2-oxo-1,2-dihydropyrazine (compound 647):

Thionylchloride (1.20 ml) was added dropwise to compound 641 (4.16 g, 13 mM) dissolved in chloroform (20 ml) under ice-cooling and stirred at room temperature for 1.5 hours. Diluted aqueous $K_2CO_3$ was added to the reaction mixture, extracted with chloroform, dried with anhydrous sodium sulfate and concentrated in vacuo to obtain the compound 647. (4.27 g, Yield: 96.9%)

Examples 125-129

1-(2-substituted ethyl)-3-p-chlorobenzyl-5,6-diethyl-2-oxo-1,2-dihydropyrazine :

Base (6 mM) and triethylamine were added to chloroethyl compound 649 1.023 g, 3 mM) dissolved in benzene (30 ml) and refluxed. Reaction mixture was poured into diluted aqueous $K_2CO_3$, waqshed and extracted the aqueous layer with benzene. Benzene layer was combined, dried with anhydrous sodium sulfate and concentrated in vacuo. Residue was charged on a column of silica-gel (C-200, 65 g) packed with chloroform and eluted with chloroform-methanol to obtain the compounds in Table 18.

A kind of base, reflux time and ratio of chloroform-methanol mixture are shown in Table 18.

Examples 130-138

1-(2-hydroxyethyl)-3-alkyl-2-oxo-1,2,5,6,7,8-hexahydroquinoxaline:

Ethylene chlorohydrin (4.03 g, 50 mM) was added to 2-hydroxy-3-alkyl-5,6,7,8-tetrahydro quinoxaline (10 mM) dissolved in aqueous 5N-NaOH (10 ml, 50 mM) and t-butanol (30 ml) and stirred at 60°C for 4 hours. Water was added to the reaction mixture, extracted with chloroform, dried with anhydrous sodium sulfate and concentrated in vacuo. Residue was charged on a column of silica-gel (C-200) packed with chloroform and eluted to obtain the compounds (hydroxy ethyl form) in Table 19.

A kind of 2-hydroxy-3-alkyl-5,6,7,8-tetrahydro quinoxaline indicated as a group $R_1$, its amount used, amount of silica-gel used in column chromatography and a kind of elution solvent are illustrated in Table 19.

Examples 139-214

1-(2-substituted ethyl)-3-alkyl-2-oxo-1,2,5,6,7,8-hexahydroquinoxaline:

Thionylchloride (1.3 equivalent) was added dropwise under ice-cooling to the hydroxy ethyl form of compound (3-5 mM), obtained in Examples 130-138, dissolved in chloroform (3-5 ml) and stirred at roomtemperature for 1 hour. Diluted aqueous $K_2CO_3$ was added to the reaction mixture, extracted 3 times with chloroform (50 ml), dried with anhydrous sodium sulfate, and concentrated in vacuo. Benzene (30ml), triethylamine (2 eq.) and base (2 eq.) were added to the residue and refluxed. Reaction mixture was poured into diluted aqueous $K_2CO_3$, then extracted with several times with benzene. Extract was dried with anhydrous sodium sulfate and concentrated in vacuo. Residue was charged on a column of silica-gel (C-200, 60 g) and eluted with chloroform-methanol (200 : 1) to obtain the compounds shown in Table 20.

Kind of starting material (hydroxy ethyl form), amount of used thereof, base and reflux time are shown in Table 20.

Example 215

1-(2-piperazinyl ethyl)-3-ethyl-5,6-dimethyl-2-oxo-1,2-dihydropyrazine:

Thionylchloride (6.6 ml) was added dropwise under ice-cooling to the compound 630 (18.72 g, 70 mM) dissolved in chloroform (70 ml) and stirred at room temperature for 1 hour. Diluted aqueous $K_2CO_3$ was added to the reaction mixture and extracted with chloroform. Extract was dried with anhydrous magnesium sulfate and concentrated in vacuo. Triethylamine (19.6 ml, 0.14 M) and anhydrous piperazine (64.4 g, 0.75 M) were added to the residue dissolved in benzene (420 ml) and refluxed for 3 hours. Reaction mixture was washed with diluted aqueous $K_2CO_3$. Further aqueous layer was extracted with benzene. Benzene layer was combined, dried with anhydrous magnesium sulfate and concentrated in vacuo. Residue was charged on a column of silica-gel (C-200, 375 g) packed with chloroform and eluted with chloroform-methanol (10 : 1) to obtain the compound 454. (13.66 g, Yield: 73.9%)

Examples 216-226

1- [2-(4′-arylpiperazinyl) ethyl] -3-ethyl-5,6-dimethyl-2-oxo-1,2-dihydropyrazine:

(a) Acid chloride method:

Triethylamine (0.84 ml, 6 mM) was added to a compound 454 (0.81 g, 3 mM) dissolved in chloroform (10 ml), and acid chloride (3 mM) was added thereto under ice-cooling. Diluted aqueous $K_2CO_3$ was added to the reaction mixture and extracted 3 times with chloroform. Combined chloroform layer was dried with anhydrous sodium sulfate and concentrated in vacuo. Residue was charged on a column of silica-gel (C-200, 80 g) packed with chloroform and eluted with chloroform-methanol to obtain the compounds shown in Table 21.

(b) Mixed anhydride method:

Triethyamine (0.70 ml) was added to the carboxylic acid (5 mM) dissolved in tetrahydrofuran (10 ml). After pivaloyl chloride (0.61 g, 5 mM) was added dropwise thereto at -5°C and stirred for 30 minutes, a chloroform sollution of compound 454 (1.06 g, 4 mM) was added dropwise. Reaction was continued under

gradually increasing up to room temperature. Reaction mixture was treated as same as of the above (a) to obtain the product shown in Table 21.

Acyaltion method, acylating agent used, reaction time and a ratio of mixture of chloroform-methanol were shown in Table 21.

Example 227

1-(2-hydroxyethyl)-3-benzyl-2-oxo-1,2,5,6,7,8-hexahydroquinoxaline (compound 643):

Ethylene chlorohydrin (16.10 g, 0.2 M) was added to 2-hydroxy-3-benzyl-5,6,7,8-tetrahydro quinoxaline (9.60 g, 40 mM) dissolved in diluted aqueous 5N-NaOH (40 ml) and t-butanol (120 ml), and stirred at 60 °C for 3 hours. A t-butanol was distilled off in vacuo. The residue was extracted 3 times with chloroform, dried with anhydrous sodium sulfate and concentrated in vacuo. Residue was charged on a column of silica-gel (C-200, 220 g) packed with chloroform, and eluted with chloroform -methanol (200 : 1) to obtain the compound 643. (10.90 g, Yield: 83.8%)

Example 228

1-(2-chloroethyl)-3-benzyl-2-oxo-1,2,5,6,7,8-hexahydroquinoxaline (compound 644):

Thionylchloride (2.96 ml, 1.3 eq.) was added dropwise under ice-cooling to the compound 643 (9.09 g, 32 mM) dissolved in chloroform (30 ml) and stirred at room temperature for 2 hours.

Diluted aqueous $K_2CO_3$ was added to the reaction mixture, extracted twice with chloroform, dried with anhydrous sodium sulfate and concentrated in vacuo. Residue was charged on a column of silica-gel (C-200, 100 g) packed with chloroform and eluted with chloroform to obtain the compound 644. (7.55 g, Yield: 78.0%)

Examples 229-234

1-(2-substituted ehtyl)-3-benzyl-2-oxo-1,2,5,6,7,8-hexahydroquinoxaline:

Base (7 mM) and triethylamine (0.98 ml) were added to a compound 644 (1.05 g, 3.5 mM) dissolved in benzene (30 ml), and refluxed. Reaction mixture was washed with diluted aqueous $K_2CO_3$ and extracted the aqueous layer with benzene. Combined benzene layer was dried with anhydrous sodium sulfate and concentrated in vacuo. Residue was charged on a column of silica-gel (C-200, 65 g) packed with chloroform and eluted with chloroform-methanol to obtain the compounds shown in Table 22.

A kind of base, reflux time and ratio of chloroform-methanol mixture are shown in Table 22.

Example 235

1-(2-hydroxyethyl)-3-(2-phenylethyl)-2-oxo-1,2,5,6,7,8-hexahydroquinoxaline (compound 645):

Ethylene chlorohydrin (9.0 g) was added to 2-hydroxy-3-(2-phenylethyl)-5,6,7,8-tetrahydro quinoxaline (5.59 g, 22 mM) dissolved in aqueous 5N-NaOH and t-butanol (60 ml), and stirred at 60 °C for 3 hours. A t-butanol was distilled off in vacuo, added water to the residue and extracted 3 times with chloroform. Extract was dried with anhydrous sodium sulfate and concentrated in vacuo. Residue was charged on a column of silica-gel (C-200, 170 g) packed with chloroform, and eluted with chloroform-methanol (200 : 1) to obtain the compound 645. (6.73 g, Yield: 90.9%)

Example 236

1-(2-chloroethyl)-3-(2-phenylethyl)-2-oxo-1,2,5,6,7,8-hexahydroquinoxaline (compound 647):

Thionylchloride (2.05 ml) was added dropwise under ice-cooling to the compound 645 (6.49 g, 22.1 mM) dissolved in chloroform (25 ml) and stirred at room temperature for 2 hours. Diluted aqueous $K_2CO_3$ was added to the reaction mixture and extracted twice with chloroform. Extract was dried with anhydrous sodium sulfate and concentrated in vacuo to obtain the compound 647, which was further used without purification for the forthcoming reaction.

Examples 237-241

1-(2-substituted ethyl)-3-(2-phenylethyl)-2-oxo-1,2,5,6,7,8-hexahydroquinoxaline:

Base (7 mM) and triethylamine (0.98 ml) were added to a compound 647 (1.11 g, 3.5 mM) dissolved in benzene (30 ml), and refluxed. Reaction mixture was washed with diluted aqueous $K_2CO_3$ and extracted

the aqueous layer with benzene. Combined benzene layer was dried with anhydrous sodium sulfate and concentrated in vacuo. Residue was charged on a column of silica-gel (C-200, 65 g) packed with chloroform and eluted with chloroform-methanol to obtain the compounds shown in Table 23.

A kind of base, reflux time and ratio of chloroform-methanol mixture are shown in Table 23.

Example 242

1-(2-hydroxyethyl)-3-ethyl-2-oxo-1,2,5,6,7,8-hexahydroquinoxaline (compound 646):

Ethylene chlorohydrin (20.2 g, 0.25 M) was added to 2-hydroxy-3-ethyl-5,6,7,8-tetrahydro quinoxaline (8.90 g, 50 mM) dissolved in diluted aqueous 5N-NaOH (50 ml) and t-butanol (150 ml), and stirred at 60°C for 3 hours. A t-butanol was distilled off in vacuo. Water was added to the residue, which was extracted 3 times with chloroform, dried with anhydrous sodium sulfate and concentrated in vacuo. Residue was charged on a column of silica-gel (C-200, 170 g) packed with chloroform, and eluted with chloroform-methanol (100 : 1) to obtain the compound 646. (9.30 g, Yield: 83.8%)

Examples 243-248

1-(2-substituted ethyl)-3-ethyl-2-oxo-1,2,5,6,7,8-hexahydroquinoxaline:

Thionylchloride (0.47 ml) was added dropwise under ice-cooling to the compound 646 (1.11 g, 5 mM) dissolved in chloroform (4 ml) and stirred at room temperature for 1 hour. Diluted aqueous $K_2CO_3$ was added to the reaction mixture and extracted with chloroform. Extract was dried with anhydrous sodium sulfate and concentrated in vacuo.

Benzene (30 ml), triethylamine (1.4 ml) and base (10 mM) were added to a residue and refluxed. Reaction mixture was poured into diluted aqueous $K_2CO_3$ and extracted with benzene. Extract was dried with anhydrous sodium sulfate and concentrated in vacuo. Residue was charged on a column of silica-gel (C-200, 65 g) packed with chloroform and eluted with chloroform-methanol to obtain the compounds shown in Table 24.

A kind of base, reflux time and ratio of chloroform-methanol mixture are shown in Table 24.

Example 249

1-(2-acetoxyethyl)-3,5,6-trimethyl-2-oxo-1,2-dihydropyrazine (compound 061):

Acetic anhydride (2 ml) was added to a compound 026 (0.91 g, 5 mM) dissolved in pyridine (10 ml) under ice-cooling and stirred at room temperature for 1.5 hour. Reaction mixture was concentrated in vacuo and diluted aqueous $K_2CO_3$ was added thereto extracted 3 times with chloroform. Extract was dried with anhydrous magnesium sulfate and concentrated in vacuo. Residue was charged on a column of silica-gel (Florisil, 60 g) packed with benzene and eluted with benzene-ethyl acetate (5 : 1) to obtain the compound 061. (0.58 g, Yield: 51.8%)

Example 250

1-(2-phenylthioethyl)-3-propyl-2-oxo-1,2,5,6,7,8-hexahydroquinoxaline (compound 615):

Thionylchloride (0.47 ml) was added dropwise under ice-cooling to the compound 614 (1.18 g, 5 mM, hydroxyethyl form) obtained in Example 47 dissolved in chloroform (5 ml) and stirred at room temperature for 1 hour. Chloroform was added to a reaction mixture, washed with diluted aqueous $K_2CO_3$ and extracted with chloroform. Combined chloroform layer was dried with anhydrous magnesium sulfate and concentrated in vacuo.

Sodium thiophenolate (0.66 g) was added to a residue dissolved in DMF (20 ml) and stirred at room temperature for 2 days. DMF was distilled off in vacuo, added diluted aqueous $K_2CO_3$ to the residue and extracted with chloroform. Extract was dried with anhydrous sodium sulfate and concentrated in vacuo.

Residue was charged on a column of silica-gel (C-200, 50 g) and eluted with benzene-ethyl acetate (5 : 1) to obtain the compound 615. (1.32 g, Yield: 80.7%)

Referential example 1

2-hydroxy-3-ethyl-5,6,7,8-tetrahydroquinoxaline:

1,2-cyclohexanedione (6.72 g, 60 mM) was added at once to a methanol solution (100 ml) of $\alpha$-aminobutylamide (5.1 g, 50 mM) at -30 °C, and aqueous 12.5 N-NaOH (5 ml) was added dropwise thereto. Reaction mixture was stirred at -30°C for 30 minutes, stopped the cooling and stirred at room temperature for 3 hours. Conc. hydrochloric acid (6.25 ml) was added to the reaction mixture under ice-cooling, furhther added sodium bicarbonate (5 g) after 10 minutes and distilled off methanol in vacuo. Residue was extracted with chloroform, washed the extracxt with water, dried with anhydrous sodium sulfate and concentrated in vacuo. Residue was washed with acetone and recrystallized from acetone to obtain the product as colorless crystals. (5.3 g. Yield: 60%), m.p.: 152 - 153 °C.

NMR ($CDCl_3$) δ (ppm): 1.25 (t, 3H, $-CH_2\underline{CH_3}$), 1.7-1.9 (m, 4H, 6-$H_2$, 7-$H_2$), 2.5-2.8 (m, 4H, 5-$H_2$, 8-$H_2$), 2.80 (q, 2H, $\underline{-CH_2}CH_3$), 13.06 (br. s, 1H, 0H)
Mass (CI): 179 ($M^+$ + 1)

Referential example 2

2-hydroxy-3-propyl-5,6,7,8-tetrahydroquinoxaline:

1,2-cyclohexanedione (5.4 g, 48 mM) was added at once to a methanol solution (80 ml) of norvalineamide hydrochloride (6.1 g, 40 mM) at -30 °C, and aqueous 12.5 N-NaOH (8 ml) was added dropwise thereto. Reaction mixture was stirred at -30°C for 30 minutes, stopped the cooling and stirred at room temperature for 3 hours. Conc. hydrochloric acid (10 ml) was added to the reaction mixture under ice-cooling, further added sodium bicarbonate (8 g) after 10 minutes and distilled off methanol in vacuo. Residue was extracted with chloroform, washed the extracxt with water, dried with anhydrous sodium sulfate and concentrated in vacuo. Residue was washed with acetone and recrystallized from acetone to obtain the product as colorless crystals. (3.8 g. Yield: 50%)
m.p.: 131 - 133°C.

NMR ($CDCl_3$)δ (ppm): 1.00 (t, 3H, $-CH_2CH_2$ $\underline{CH_3}$), 1.55-1.90 (m, 4H, 6-H, $-CH_2CH_2CH_3$, 6-$H_2$, 7-$H_2$), 2.67-2.83 (m, 6H, $\underline{-CH_2}CH_2CH_3$, 5-$H_2$, 6-$H_2$), 13.09 (br. s, 1H, 0H)
Mass (CI): 193 ($M^+$ + 1)

Referential examples 3-13:

2-hydroxy-3-alkyl-5,6,7,8-tetrahydroquinoxaline:

1,2-cyclohexanedione (6.72 g, 60 mM) was added at once to a methanol solution (100 ml) of α-amino acidamide hydro chloride (50 mM) at -30 °C, and aqueous 12 N-NaOH (10 ml) was added dropwise thereto. Reaction mixture was stirred for 30 minutes, stopped the cooling and stirred at room temperature for 5 hours. Conc. hydrochloric acid (12.5 ml) was added to the reaction mixture under ice-cooling, furhther added sodium bicarbonate (10 g) after 10 minutes and distilled off methanol in vacuo. Residue was extracted with chloroform, washed the extracxt with water, dried with anhydrous sodium sulfate and concentrated in vacuo. Residue was washed with acetone and recrystallized from acetone to obtain the product shown in Table 25.

A kind of product, yield (weight and %) and physical properties are shown in Table 25.

Referential example 14

2-hydroxy-3-benzyl-5,6,7,8-tetrahydroquinoxaline:

A methanol (30 ml) solution of 1,2-cyclohexanedione (13.44 g, 0.12 M) was added to phenylalanineamide hydrochloride (20.05 g, 0.1 M) dissolved in methanol (200 ml) under cooling below -30 °C, and aqueous 12.5 N-NaOH (20 ml) was added dropwise thereto. Reaction mixture was stirred under - 30 °C for 30 minutes, stopped the cooling and stirred at room temperature for 3 hours. Conc. hydrochloric acid (25 ml) was added to the reaction mixture under ice-cooling, furhther added sodium bicarbonate (15 g) after 10 minutes stirring and distilled off methanol in vacuo. Residue, which was added with water, was extracted 3 times with chloroform, washed the extracxt with water, dried with anhydrous magnesium sulfate and concentrated in vacuo. Residue was washed with acetone and recrystallized from acetone to obtain the product. (19.7 g. Yield: 82.1%)

NMR ($CDCl_3$, TMS) δ (ppm): 1.6-2.0 (m, 4H), 2.4-2.7 (m, 4H), 4.02 (s, 2H), 7.0-7.4 (m, 5H)
Mass (CI): 241 ($M^+$ + 1)

Referential example 15

2-hydroxy-3-(phenylethyl)-5,6,7,8-tetrahydroquinoxaline:

A methanol (20 ml) solution of 1,2-cyclohexanedione (5.38 g) was added to a methanol suspention (100 ml) of α-amino-(3-phenyl)-butylamide hydrochloride (8.58 g, 40 mM) at below -30 °C, and aqueous 12.5 N-NaOH (8 ml) was added dropwise thereto. Reaction mixture was stirred at below -30 °C for 30 minutes, stopped the cooling and stirred at room temperature for 6 hours. Conc. hydrochloric acid (8 ml) was added to the reaction mixture under ice-cooling, furhther added sodium bicarbonate (6.0 g) after 10 minutes stirring and distilled off methanol in vacuo. Residue, which was added with water, was extracted 3 times

with chloroform, washed the extracxt with water, dried with anhydrous sodium sulfate and concentrated in vacuo. Residue was washed with acetone and recrystallized from acetone to obtain the product. (6.79 g. Yield: 66.8%)

NMR ($CDCl_3$, TMS) δ (ppm): 1.6-2.0 (m, 4H), 2.5-2.8 (m, 4H), 3.06 (s, 2H x 2), 7.1-7.3 (s, 5H), 12.9 (br. s, 1H)

Mass (CI): 255 ($M^+$ + 1)

Referential examples 16-23:

2-hydroxy-3-alkyl-5,6-diethylpyrazine:

α-amino acidamide (0.20 M) and 3,4-hexane (22.8 g, 0.20 M) were added to triethylamine (200 ml), stirred at room temperature for 30 minutes and refluxed for 15 hours. Triethyamine was distilled off in vacuo, dissolved a residue in chloroform and washed with diluted aqueous $K_2CO_3$. Chloroform layer was dried with anhydrous sodium sulfate and concentrated in vacuo. Residue was recrystallized from acetone to obtain the products shown in Table 26.

Product, yield (weight and %) and physical properties are shown in Table 26.

Table 6 1－(2－substituted ethyl)－3, 5, 6－trimethyl－2－oxo－1, 2－dihidropyrazine

| Example | Product * | Base | Starting Material * | Heat time (hr) | Elution solvent ratio | Yield (g) | Yield (%) |
|---|---|---|---|---|---|---|---|
| 2 | 018 | 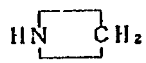 | 026 | 1.75h | 20:1 | 1.96 | 78.5 |
| 3 | 019 | 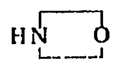 | 026 | 4 | 40:1 | 1.61 | 64.1 |
| 4 | 020 | 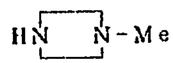 | 026 | 1.75 | 20:1 | 1.70 | 64.4 |
| 5 | 021 | 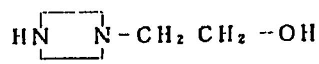 | 026 | 2 | 10:1 | 2.25 | 76.5 |
| 6 | 022 | 1－imidazole | 026 | 2 | 50:1 | 1.45 | 62.4 |
| 7 | 023 | 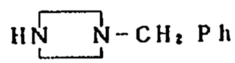 | 026 | 5 | 100:1 | 2.42 | 71.2 |
| 8 | 024 | 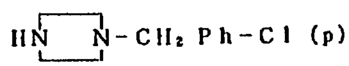 | 026 | 5 | 100:1 | 3.34 | 86.5 |
| 9 | 025 | 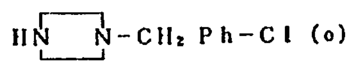 | 026 | 5 | 100:1 | 3.64 | 97.2 |

*; Compound No.

Table 7 1－(2－substituted thioethyl)－3, 5, 6－trimethy－2－oxo－1, 2－dihydropyrazine

| Exapmle | Product * | Mercaptane | Starting material * | Yield (g) | Yield (%) |
|---|---|---|---|---|---|
| 10 | 027 | HS－Ph | 026 | 1.06 | 77.4 |
| 11 | 028 | 1－methyl－5－mercaptotetrazole | 026 | 1.04 | 78.8 |

Table 8 1-(2-substituted ethyl)-3-phenyl-5, 6-dimethyl-2-oxo-1, 2-dihydropyrazine

| Example | Product * | Base | Starting material * | Heat time (hr) | Elution solvent ratio | Yield (g) | Yield (%) |
|---|---|---|---|---|---|---|---|
| 16 | 110 | 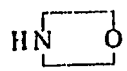 | 612 | 3 | 100:1 | 0.70 | 89.5 |
| 17 | 111 | 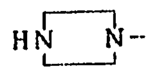 $-CH_2Ph-Cl$ (p) | 612 | 3 | 100:1 | 0.99 | 91.0 |

* ; Compound No.

Table 9 1-(2-substituted ethyl)-3-benzyl-5, 6-dimethyl 2-oxo-1, 2-dihydropyrazine

| Example | Product * | Base | Starting material * | Heat time (hr) | Elution solvent ratio | Yield (g) | Yield (%) |
|---|---|---|---|---|---|---|---|
| 20 | 113 | 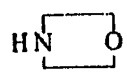 | 613 | 3 h | 100:1 | 0.78 | 79.5 |
| 21 | 114 | 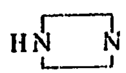 $-CH_2Ph$ | 613 | 2 | 100:1 | 1.07 | 85.5 |
| 22 | 115 | 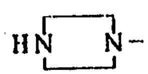 $-CH_2Ph-Cl$ (p) | 613 | 3 | 100:1 | 0.78 | 57.7 |
| 23 | 116 | 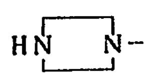 $-CH_2Ph-Cl$ (o) | 613 | 2 | 100:1 | 0.96 | 71.0 |

* ; Compound No.

Table 10 1-(2-hydroxyethyl)-3-alkyl-5,6-dimetyl-2-oxo-1,2-dihydropyrazine

| Example | Product * | Starting material ($R_1$) | | Reaction time (hr) | Elution solvent ratio | Yield (g) | Yield (%) |
|---|---|---|---|---|---|---|---|
| 24 | 616 | Pro | 3.32 g (20 mM) | 2 h | 100:1 | 3.42 | 81.4 |
| 25 | 622 | iso-Pro | 4.50 g (27 mM) | 2 | 100:1 | 4.32 | 76.2 |
| 26 | 624 | Bu | 2.70 g (15 mM) | 2 | 50:1 | 3.31 | 98.5 |
| 27 | 617 | iso-Bu | 5.40 g (30 mM) | 2 | 50:1 | 5.12 | 76.2 |
| 28 | 623 | sec-Bu | 4.50 g (25 mM) | 2 | 100:1 | 4.74 | 84.6 |

* ; Compound No.

Table 11 1-(2-substituted ethyl)-3-alkyl-5,6-dimethyl-2-oxo-1,2-dihydropyrazine

| Example | Product * | Base | Starting material * | Heat time (hr) | Elution solvent ratio | Yield (g) | Yiled (%) |
|---|---|---|---|---|---|---|---|
| 29 | 121 | 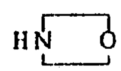 | 616 | 1.5 h | 100:1 | 0.94 | 67.4 |
| 30 | 122 | 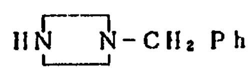 | 616 | 2 | 100:1 | 1.00 | 67.9 |
| 31 | 123 | 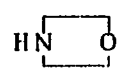 | 617 | 2.5 | 150:1 | 0.72 | 61.4 |
| 32 | 124 | 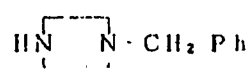 | 617 | 2 | 150:1 | 1.41 | 92.3 |

Table 11 ( Continued )

| | | | | | | | |
|---|---|---|---|---|---|---|---|
| 33 | 125 | 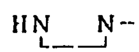 $-CH_2Ph-Cl$ (p) | 617 | 1.5 | 150:1 | 1.58 | 94.8 |
| 34 | 126 | 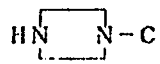 $-CH_2Ph-Cl$ (m) | 617 | 1.5 | 150:1 | 1.27 | 76.2 |
| 35 | 619 | 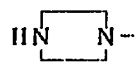 $-CH_2Ph-F$ (p) | 617 | 1.5 | 150:1 | 1.16 | 72.5 |
| 36 | 148 | 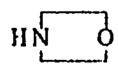 | 622 | 1.5 | 200:1 | 0.94 | 84.2 |
| 37 | 149 | 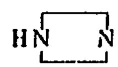 $-CH_2Ph$ | 622 | 1.5 | 200:1 | 1.42 | 96.5 |
| 38 | 150 | 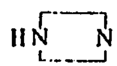 $-CH_2Ph-Cl$ (p) | 622 | 2 | 200:1 | 1.41 | 87.6 |
| 39 | 151 | 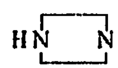 $-CH_2Ph-F$ (p) | 622 | 2 | 150:1 | 1.21 | 78.4 |
| 40 | 156 | 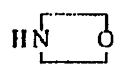 | 624 | 1.5 | 150:1 | 0.85 | 72.5 |
| 41 | 157 | 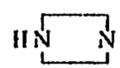 $-CH_2Ph$ | 624 | 1.5 | 150:1 | 1.03 | 67.4 |
| 42 | 158 | 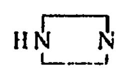 $-CH_2Ph-Cl$ (p) | 624 | 1.5 | 150:1 | 1.08 | 64.8 |
| 43 | 152 | 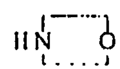 | 623 | 1 | 150:1 | 0.49 | 41.8 |

Table 11 ( Continued )

| | | | | | | | |
|---|---|---|---|---|---|---|---|
| 44 | 153 | 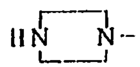 $-CH_2Ph$ | 623 | 1.25 | 150:1 | 0.64 | 41.9 |
| 45 | 154 | 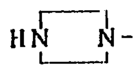 $-CH_2Ph-Cl$ (p) | 623 | 1.25 | 150:1 | 1.05 | 63.0 |
| 46 | 155 | 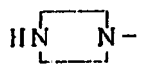 $-CH_2Ph-F$ (p) | 623 | 1.5 | 150:1 | 1.12 | 70.0 |

* ; Compound No.

Table 12 1-(2-hydroxyethyl)-3-alkyl-2-oxo-1, 2, 5, 6, 7, 8-hexahydro quinoxaline

| Example | Product * | Starting material ($R_1$) | | Reaction time (hr) | Elution solvent ratio | Yield (g) | Yield (%) |
|---|---|---|---|---|---|---|---|
| 47 | 614 | Pro | 6.26g (32mM) | 1.25h | 50:1 | 7.15 | 94.7 |
| 48 | 621 | Bu | 5.15g (25mM) | 2 | 50:1 | 6.15 | 98.4 |
| 49 | 618 | iso-Bu | 4.12g (20mM) | 2 | 50:1 | 4.12 | 82.4 |

* ; Compound No.

Table 13 1-(2-substituted ethyl)-3-alkyl-2-oxo-1, 2, 5, 6, 7, 8-hexahydro quinoxaline

| Example | Product* | Base | SAtarting material* | Heat time (hr) | Elution solvent ratio | Yield (g) | Yield (%) |
|---|---|---|---|---|---|---|---|
| 50 | 118 | 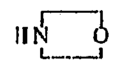 | 614 | 3 h | 100:1 | 1.14 | 93.5 |
| 51 | 119 | 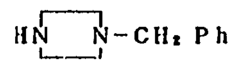 | 614 | 3 | 100:1 | 1.21 | 95.8 |
| 52 | 120 | 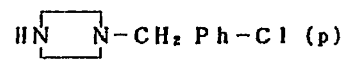 | 614 | 2.5 | 100:1 | 1.57 | 91.9 |
| 53 | 117 | 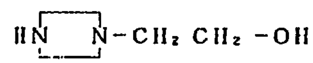 | 614 | 2.5 | 50:1 | 0.70 | 62.2 |
| 54 | 127 | 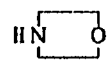 | 618 | 2.5 | 200.1 | 1.07 | 83.6 |
| 55 | 128 | 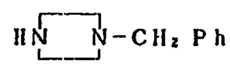 | 618 | 1 | 200:1 | 1.58 | 96.8 |
| 56 | 129 | 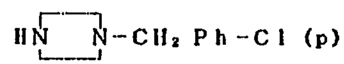 | 618 | 1 | 200:1 | 1.46 | 82.5 |
| 57 | 141 | 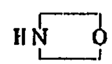 | 621 | 1 | 200:1 | 0.76 | 59.6 |
| 58 | 142 | 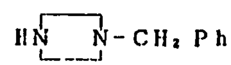 | 621 | 1 | 200:1 | 0.95 | 58.2 |
| 59 | 143 | 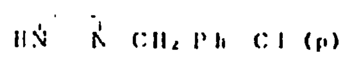 | 621 | 2 | 200:1 | 1.49 | 84.2 |
| 60 | 144 | 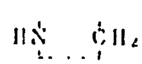 | 621 | 1 | 200:1 | 0.86 | 67.8 |

Table 13 ( continued )

| | | | | | | | |
|---|---|---|---|---|---|---|---|
| 61 | 145 | HN N-$CH_2CH_2$-OH | 621 | 2 | 50:1 | 0.92 | 63.5 |
| 62 | 146 | HN N-$CH_2$Ph-F (p) | 621 | 1.25 | 200:1 | 1.15 | 67.5 |

* ; Compound No.

Table 14 1-(2-hydroxy ethyl)-3-alkyl-5, 6-diethyl-2-oxo-1, 2-dihydropyrazine

| Example | Product* | Starting material ( $R_1$ ) | | Reaction time ( hr ) | Elution solvent ration | Yield ( g ) | Yiled ( % ) |
|---|---|---|---|---|---|---|---|
| 63 | 620 | Pro | 7.76g (40mM) | 5.5h | 50:1 | 6.89 | 73.3 |
| 64 | 625 | iso-Pro | 9.70g (50mM) | 5.5 | 100:1 | 8.70 | 73.1 |
| 65 | 628 | Bu | 10.40g (50mM) | 3 | 100:1 | 8.53 | 68.2 |
| 66 | 626 | iso-Bu | 5.20g (25mM) | 3 | 100:1 | 5.08 | 81.3 |
| 67 | 627 | sec-Bu | 10.40g (50mM) | 3 | 100:1 | 9.94 | 79.5 |
| 68 | 629 | Et | 4.50g (25mM) | 2 | 100:1 | 4.38 | 78.2 |

* ; Compound No.

TABLE 15 1 - (2 -substituted ethyl ) - 3 -alkyl - 5, 6 -diethyl - 2 -oxo - 1, 2 -dihydropyrazine

| Example | Product * | Base | Starting material * | Heat time ( hr ) | Elution Solvent ratio | Yield ( g ) | Yield ( % ) |
|---|---|---|---|---|---|---|---|
| 69 | 130 | 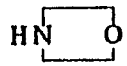 | 620 | 3 h | 100:1 | 0.77 | 62.7 |
| 70 | 131 | 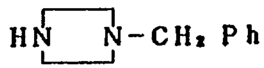 | 620 | 1.5 | 200:1 | 1.05 | 66.3 |
| 71 | 132 | 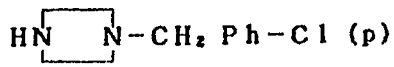 | 620 | 1.25 | 200:1 | 0.90 | 52.1 |
| 72 | 133 | 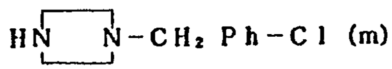 | 620 | 1.5 | 200:1 | 1.02 | 59.0 |
| 73 | 134 | 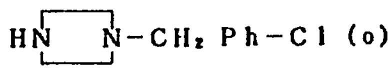 | 620 | 1.25 | 200:1 | 0.63 | 36.5 |
| 74 | 159 | 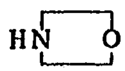 | 625 | 1.25 | 200:1 | 0.65 | 52.9 |
| 75 | 160 | 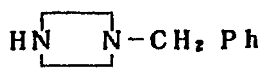 | 625 | 1.25 | 200:1 | 0.74 | 46.8 |
| 76 | 161 | 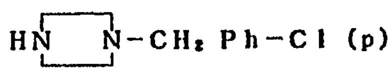 | 625 | 1.25 | 200:1 | 0.99 | 57.5 |
| 77 | 162 | 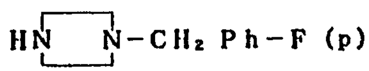 | 625 | 1.25 | 200:1 | 0.55 | 33.2 |

Table 15 ( continued )

| | | | | | | | |
|---|---|---|---|---|---|---|---|
| 78 | 163 | 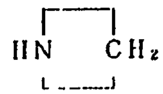 | 625 | 1. 25 | 200:1 | 0. 55 | 45. 1 |
| 79 | 164 | 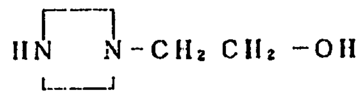 | 625 | 1. 25 | 50:1 | 0. 47 | 33. 6 |
| 80 | 165 | 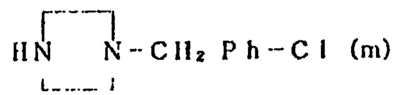 | 625 | 1. 25 | 200:1 | 0. 77 | 44. 7 |
| 81 | 166 | 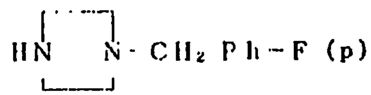 | 626 | 1. 25 | 200:1 | 1. 20 | 70. 1 |
| 82 | 167 | 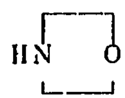 | 626 | 1. 25 | 200:1 | 0. 70 | 54. 5 |
| 83 | 168 | 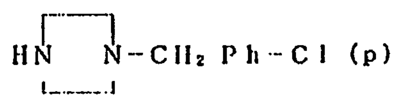 | 626 | 1. 25 | 200:1 | 1. 17 | 65. 6 |

Table 15 ( Continued )

| | | | | | | | | |
|---|---|---|---|---|---|---|---|---|
| 84 | 170 | 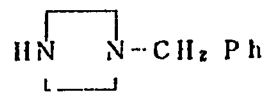 | 626 | 1.25 | 200:1 | 1.31 | 79.9 |
| 85 | 171 | 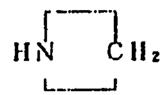 | 627 | 1.25 | 200:1 | 0.33 | 25.9 |
| 86 | 172 | 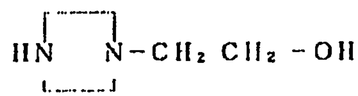 | 627 | 2 | 50:1 | 0.30 | 20.6 |
| 87 | 173 | 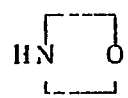 | 627 | 1 | 200:1 | 0.68 | 53.0 |
| 88 | 174 | 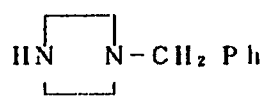 | 627 | 1 | 200:1 | 0.46 | 28.0 |
| 89 | 175 | 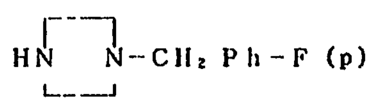 | 627 | 1.25 | 200:1 | 0.39 | 22.8 |

Table 15 ( Continued )

| | | | | | | | |
|---|---|---|---|---|---|---|---|
| 90 | 176 | 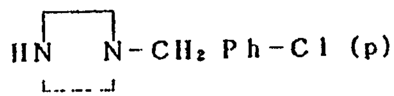 | 627 | 1 | 200:1 | 0.78 | 43.6 |
| 91 | 177 | 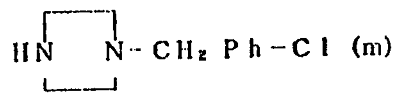 | 627 | 1.25 | 200:1 | 0.52 | 29.1 |
| 92 | 178 | 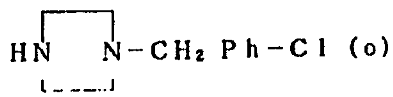 | 628 | 1.25 | 200:1 | 1.08 | 60.8 |
| 93 | 179 | 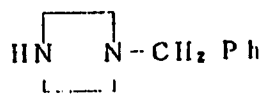 | 628 | 1.25 | 200:1 | 0.92 | 56.1 |
| 94 | 180 | 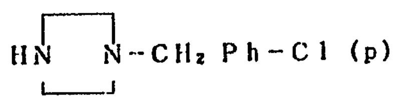 | 628 | 1.25 | 200:1 | 1.54 | 86.7 |
| 95 | 181 | 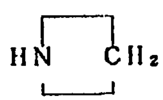 | 628 | 1 | 200:1 | 0.90 | 70.5 |

Table 15 ( Continued )

| | | | | | | | |
|---|---|---|---|---|---|---|---|
| 96 | 182 | 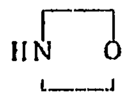 | 628 | 1. 25 | 200:1 | 0. 98 | 76. 3 |
| 97 | 183 | 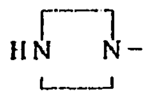 $-CH_2Ph-F$ (p) | 628 | 1. 25 | 200:1 | 1. 28 | 74. 8 |
| 98 | 185 | 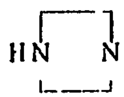 $-CH_2Ph-Cl$ (m) | 628 | 1. 25 | 200:1 | 0. 78 | 43. 9 |
| 99 | 187 | 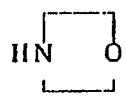 | 629 | 2 | 200:1 | 0. 86 | 77. 6 |
| 100 | 188 | 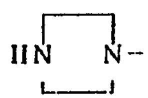 $-CH_2Ph-Cl$ (p) | 629 | 1. 25 | 100:1 | 0. 80 | 48. 1 |
| 101 | 189 | 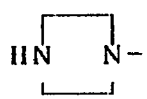 $-CH_2Ph$ | 629 | 1. 25 | 100:1 | 0. 79 | 51. 7 |

* ; Compound No.

Table 16 1-(2-substituted ethyl)-3-ethyl-5, 6-dimethyl-2-oxo-1, 2-dihydropyrazine

| Example | Product* | Base | Starting material* | Heat time (hr) | Elution solvent ration | Yield (g) | Yield (%) |
|---|---|---|---|---|---|---|---|
| 103 | 190 | 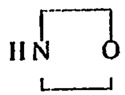 | 630 | 2 | 200:1 | 1.03 | 77.7 |
| 104 | 191 | 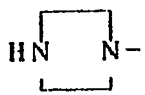 $-CH_2Ph-Cl$ (p) | 630 | 2 | 200:1 | 1.54 | 79.3 |
| 105 | 192 | 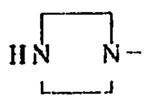 $-CH_2Ph-F$ (p) | 630 | 1.5 | 200:1 | 1.23 | 66.1 |
| 106 | 193 | 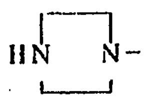 $-CH_2Ph-OMe$ (p) | 630 | 2 | 200:1 | 1.70 | 88.5 |
| 107 | 287 | 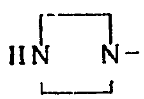 $-CH_2Ph$ | 630 | 1 | 200:1 | 1.37 | 77.4 |

Table 16 ( Continued )

| | | | | | | | |
|---|---|---|---|---|---|---|---|
| 108 | 288 | 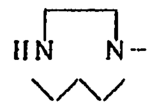 $-CH_2Ph-Cl$ (p) | 630 | 1 | 200:1 | 1.42 | 70.6 |
| 109 | 289 | 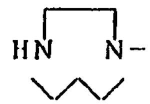 $-CH_2Ph-F$ (p) | 630 | 1 | 200:1 | 1.05 | 54.4 |
| 110 | 290 | 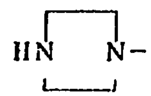 $-CH_2Ph-NO_2$ (p) | 630 | 1.5 | 200:1 | 1.39 | 69.7 |

* Compound No.

Table 17 1 − ( 2-substitutedted ethyl ) − 3 − benzyl − 5, 6 − diethyl − 2 − oxo − 1, 2 − dihydropyrazine

| Example | Product * | Base | Starting material * | Heat time ( hr ) | Elution solvent ratio | Yield ( g ) | Yield ( % ) |
|---|---|---|---|---|---|---|---|
| 112 | 291 | 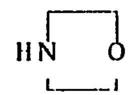 | 640 | 4 h | 100 : 1 | 1. 15 | 81. 0 |
| 113 | 292 | 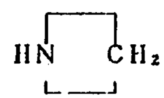 | 640 | 1 | 100 : 1 | 1. 15 | 81. 4 |
| 114 | 293 | 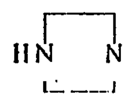 $-CH_2Ph$ | 640 | 3 | 100 : 1 | 1. 11 | 62. 5 |
| 115 | 294 | 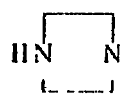 $-CH_2Ph-Cl$ (p) | 640 | 2 | 100 : 1 | 1. 60 | 83. 6 |
| 116 | 295 | 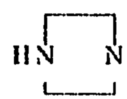 $-CH_2Ph-Cl_2$ (2, 4) | 640 | 2. 5 | 100 : 1 | 0. 95 | 46. 3 |

Table 17 ( Continued )

| | | | | | | | |
|---|---|---|---|---|---|---|---|
| 117 | 296 | 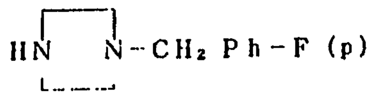 | 640 | 4 | 100:1 | 1.45 | 78.5 |
| 118 | 297 | 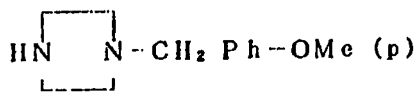 | 640 | 2.5 | 100:1 | 0.99 | 52.2 |
| 119 | 374 | 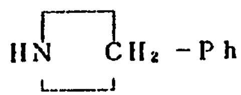 | 640 | 1 | 200:1 | 1.21 | 70.5 |
| 120 | 375 | 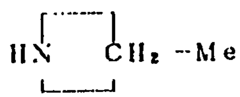 | 640 | 1 | 200:1 | 0.99 | 67.4 |
| 121 | 378 | 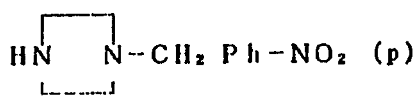 | 640 | 1 | 100:1 | 1.57 | 80.3 |
| 122 | 380 | 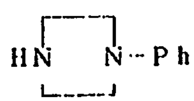 | 640 | 2 | 200:1 | 0.73 | 42.4 |

* ; Compound No.

Table 18 1 - (2-substituted ethyl) - 3 - p-chlorobenzyl - 5, 6-diethyl - 2-oxo - 1, 2-dihydropyrazine

| Example | Product * | Base | Starting material * | Heat time (hr) | Elution solvent ratio | Yield (g) | Yield (%) |
|---|---|---|---|---|---|---|---|
| 125 | 384 | 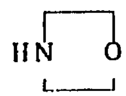 | 641 | 2 | 100:1 | 0.89 | 76.2 |
| 126 | 467 | 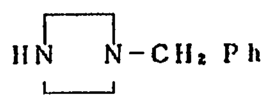 | 641 | 2 | 100:1 | 1.10 | 76.6 |
| 127 | 385 | 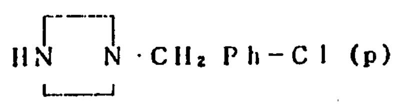 | 641 | 2 | 100:1 | 1.23 | 79.9 |
| 128 | 386 | 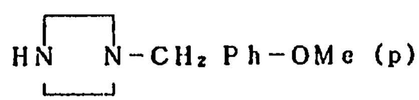 | 641 | 2 | 100:1 | 1.15 | 75.4 |
| 129 | 387 | 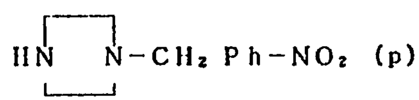 | 641 | 2 | 100:1 | 1.03 | 56.2 |

*; Compound No.

Table 19 1 − (2 −hydroxyethyl) − 3 −alkyl − 2 −oxo − 1, 2, 5, 6, 7, 8 −hexyahydro quinozaline

| Example | Product* | Starting materil ($R_1$) | | silica-gel | Elution solvent | | Yield (g) | Yield(%) |
|---|---|---|---|---|---|---|---|---|
| 130 | 631 | $-(CH_2)_4CH_3$ | 30mM | 150g | chloroform - methanol | (200 : 1) | 6. 44 | 81. 3 |
| 131 | 632 | $-(CH_2)_5CH_3$ | 20mM | 100g | chloroform - methanol | (200 : 1) | 4. 66 | 83. 8 |
| 132 | 633 | $-(CH_2)_6CH_3$ | 30mM | 150g | chloroform - methanol | (200 : 1) | 8. 39 | 95. 8 |
| 133 | 634 | $-(CH_2)_7CH_3$ | 28mM | 150g | chloroform - methanol | (200 : 1) | 7. 60 | 89. 2 |
| 134 | 635 | $-(CH_2)_8CH_3$ | 40mM | 200g | chloroform - methanol | (200 : 1) | 11. 00 | 85. 9 |
| 135 | 636 | $-(CH_2)_9CH_3$ | 30mM | 150g | chloroform - methanol | (200 : 1) | 8. 42 | 84. 0 |

Table 19 ( Continued )

| | | | | | | | |
|---|---|---|---|---|---|---|---|
| 136 | 637 | $-(CH_2)_{11}CH_3$ | 20 mM | 100 g | benzene - ethyl acetate (5 : 1→3 : 1) | 6. 06 | 84. 2 |
| 137 | 638 | $-(CH_2)_{13}CH_3$ | 15 mM | 100 g | benzene - ethyl acetate (5 : 1) | 3. 86 | 66. 2 |
| 138 | 639 | $-(CH_2)_{15}CH_3$ | 20 mM | 100 g | benzene - ethyle acetate (5 : 1→3 : 1) | 5. 92 | 70. 8 |

* ; Compound No.

Table 20 1-(2-substituted ethyl)-3-alkyl-2-oxo-1, 2, 5, 6, 7, 8-hexahydro quinoxaline

| Example | Product* | Starting material* | Base | Heat time ( hr ) | Yield ( g ) | Yield ( % ) |
|---|---|---|---|---|---|---|
| 139 | 250 | 631 1. 06 g (4 mM) | 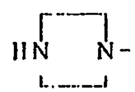 $-CH_2$ Ph-Cl (p) | 1. 5 h | 1. 59 | 87. 1 |
| 140 | 251 | 631 1. 06 g (4 mM) | 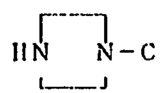 $-CH_2$ Ph-F (p) | 2 | 1. 65 | 93. 8 |

Table 20 ( Continued )

| | | | | | | | |
|---|---|---|---|---|---|---|---|
| 141 | 252 | 631 | 1. 32g (5mM) | 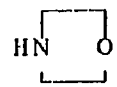 | 1. 5 | 1. 05 | 63. 1 |
| 142 | 253 | 631 | 1. 06g (4mM) | 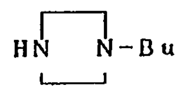 | 2 | 1. 01 | 65. 1 |
| 143 | 254 | 631 | 1. 06g (4mM) | 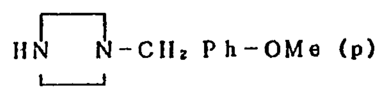 | 1. 5 | 1. 35 | 74. 7 |
| 144 | 255 | 632 | 1. 25g (4. 5mM) | 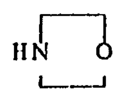 | 2 | 1. 34 | 85. 8 |
| 145 | 256 | 632 | 0. 95g (3. 5mM) | 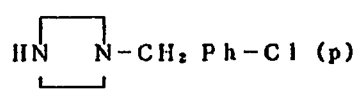 | 2 | 0. 88 | 53. 4 |
| 146 | 257 | 632 | 0. 95g (3. 5mM) | 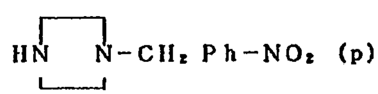 | 2 | 1. 50 | 89. 1 |

Table 20 ( Continued )

| | | | | | | |
|---|---|---|---|---|---|---|
| 147 | 258 | 632 1.12g (4mM) | 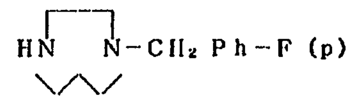 | 2 | 1.44 | 76.9 |
| 148 | 305 | 632 1.12g (4mM) | 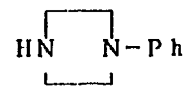 | 2 | 1.38 | 81.7 |
| 149 | 306 | 632 1.12g (4mM) | 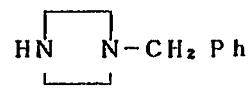 | 2 | 1.32 | 75.7 |
| 150 | 307 | 632 1.12g (4mM) | 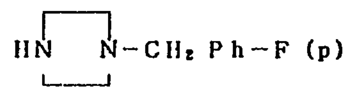 | 2 | 1.49 | 83.9 |
| 151 | 308 | 632 1.12g (4mM) | 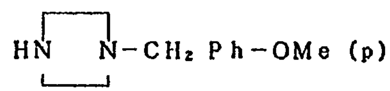 | 2 | 1.73 | 92.8 |
| 152 | 309 | 632 1.12g (4mM) | 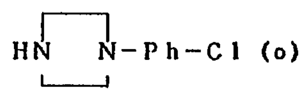 | 2.5 | 1.38 | 75.6 |

Table 20 ( Continued )

| | | | | | | | |
|---|---|---|---|---|---|---|---|
| 153 | 310 | 632 | 1.12g (4mM) | 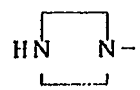 -Ph-OMe (o) | 2.5 | 1.43 | 79.1 |
| 154 | 311 | 632 | 1.12g (4mM) | 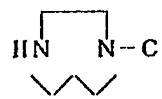 -$CH_2$ Ph-$NO_2$ (p) | 2.5 | 1.44 | 72.7 |
| 155 | 312 | 632 | 1.12g (4mM) | 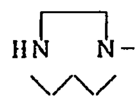 -$CH_2$ Ph-Cl (p) | 2 | 1.49 | 76.9 |
| 156 | 313 | 632 | 1.12g (4mM) | 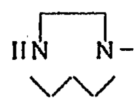 -$CH_2$ Ph-OMe (p) | 2 | 0.81 | 42.2 |
| 157 | 259 | 633 | 1.17g (4mM) | 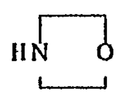 | 2 | 0.94 | 65.1 |

Table 20 ( Continued )

| | | | | | | |
|---|---|---|---|---|---|---|
| 158 | 260 | 633 1.17g (4mM) | 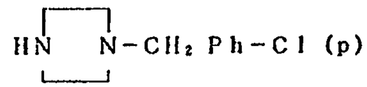 | 2 | 1.03 | 53.4 |
| 159 | 261 | 633 1.17g (4mM) | 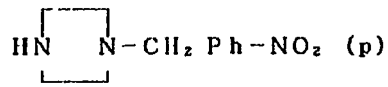 | 2 | 1.50 | 75.8 |
| 160 | 262 | 633 1.17g (4mM) | 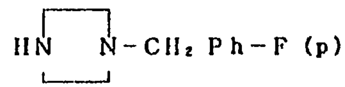 | 2 | 1.28 | 68.4 |
| 161 | 263 | 633 1.17g (4mM) | 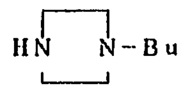 | 2 | 0.85 | 51.1 |
| 162 | 264 | 633 1.17g (4mM) | 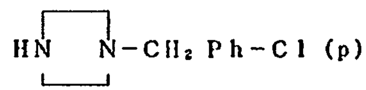 | 2 | 1.42 | 71.4 |
| 163 | 314 | 633 1.17g (4mM) | 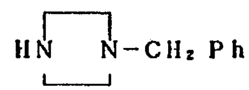 | 2 | 1.50 | 83.3 |
| 164 | 315 | 633 1.17g (4mM) | 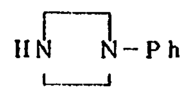 | 2 | 0.44 | 25.2 |

Table 20 ( Continued )

| | | | | | | | |
|---|---|---|---|---|---|---|---|
| 165 | 316 | 633 | 1.17g (4mM) | 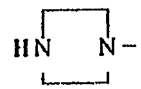 -Ph-OMe (o) | 2 | 1.70 | 93.3 |
| 166 | 317 | 633 | 1.17g (4mM) | 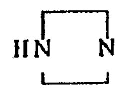 -Ph-Cl (o) | 2 | 1.30 | 69.1 |
| 167 | 318 | 633 | 1.17g (4mM) | 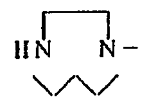 $-CH_2$ Ph-F (p) | 2.5 | 1.11 | 57.6 |
| 168 | 319 | 633 | 1.17g (4mM) | 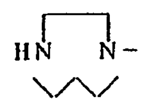 $-CH_2$ Ph-OMe (p) | 2 | 1.01 | 51.1 |
| 169 | 320 | 633 | 1.17g (4mM) | 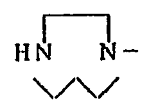 $-CH_2$ Ph-$NO_2$ (p) | 1.5 | 1.29 | 63.4 |
| 170 | 265 | 634 | 1.23g (4mM) | 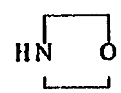 | 2 | 1.01 | 67.3 |
| 171 | 266 | 634 | 0.92g (3mM) | 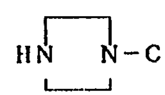 $-CH_2$ Ph-Cl (p) | 2 | 1.49 | 99.6 |

Table 20 ( Continued )

| | | | | | | | |
|---|---|---|---|---|---|---|---|
| 172 | 267 | 634 | 0. 92g (3mM) | 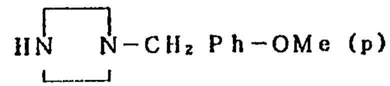 | 2 | 1. 24 | 83. 7 |
| 173 | 268 | 634 | 0. 92g (3mM) | 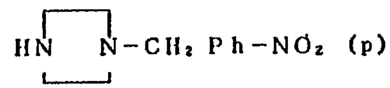 | 2 | 1. 40 | 91. 7 |
| 174 | 269 | 634 | 0. 92g (3mM) | 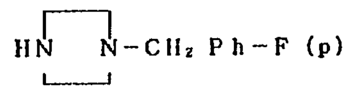 | 2 | 1. 27 | 87. 8 |
| 175 | 270 | 634 | 1. 23g (4mM) | 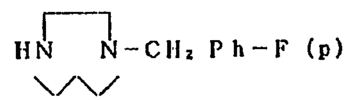 | 2 | 1. 18 | 59. 5 |
| 176 | 321 | 634 | 1. 23g (4mM) | 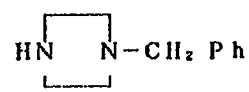 | 2 | 1. 79 | 96. 4 |
| 177 | 322 | 634 | 1. 23g (4mM) | 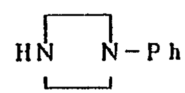 | 2 | 1. 58 | 87. 8 |
| 178 | 323 | 634 | 1. 23g (4mM) | 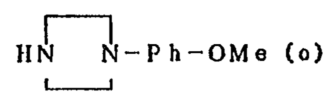 | 3 | 1. 90 | 99. 0 |

Table 20 ( Continued )

| | | | | | | | |
|---|---|---|---|---|---|---|---|
| 179 | 324 | 634 | 1.23g (4mM) | 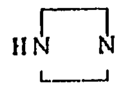 -Ph-Cl (o) | 2 | 1.54 | 79.5 |
| 180 | 325 | 634 | 1.23g (4mM) | 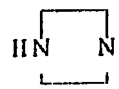 -$CH_2$Ph-$NO_2$ (p) | 2 | 1.45 | 69.3 |
| 181 | 326 | 634 | 1.23g (4mM) | 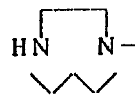 -$CH_2$Ph-OMe (p) | 2 | 1.33 | 65.5 |
| 182 | 327 | 634 | 1.23g (4mM) | 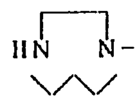 -$CH_2$Ph-Cl (p) | 2 | 1.41 | 68.8 |
| 183 | 271 | 635 | 1.28g (4mM) | 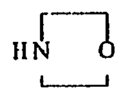 | 2 | 1.03 | 66.2 |
| 184 | 272 | 635 | 0.96g (3mM) | 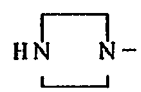 -$CH_2$Ph-Cl (p) | 2 | 1.15 | 74.8 |
| 185 | 273 | 635 | 0.96g (3mM) | 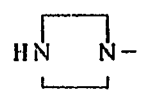 -$CH_2$Ph-F (p) | 2 | 1.25 | 84.0 |

Table 20 ( Continued )

| | | | | | | | |
|---|---|---|---|---|---|---|---|
| 186 | 274 | 635 | 0. 96g (3mM) | 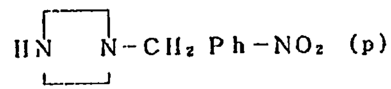 | 2 | 1. 15 | 73. 3 |
| 187 | 275 | 635 | 0. 96g (3mM) | 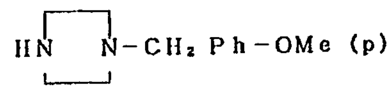 | 2 | 0. 72 | 47. 2 |
| 188 | 328 | 635 | 0. 96g (3mM) | 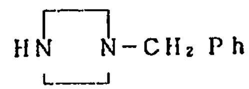 | 2 | 1. 30 | 90. 7 |
| 189 | 329 | 635 | 0. 96g (3mM) | 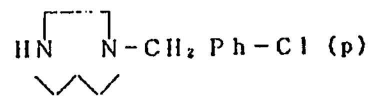 | 2 | 0. 87 | 55. 1 |
| 190 | 330 | 635 | 0. 96g (3mM) | 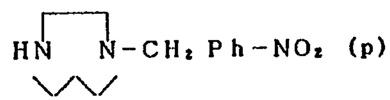 | 2. 5 | 1. 08 | 67. 0 |
| 191 | 331 | 635 | 0. 96g (3mM) | 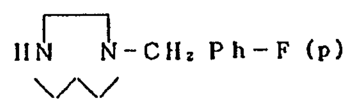 | 2 | 0. 61 | 39. 9 |
| 192 | 332 | 635 | 0. 96g (3mM) | 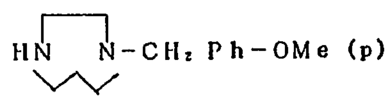 | 2 | 1. 04 | 66. 4 |

Table 20 ( Continued )

| | | | | | | |
|---|---|---|---|---|---|---|
| 193 | 334 | 636 1.01g (3mM) | 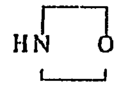 | 2 | 0.96 | 79.4 |
| 194 | 335 | 636 1.01g (3mM) | 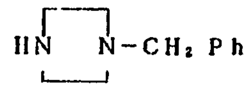 | 2 | 1.28 | 86.6 |
| 195 | 336 | 636 1.01g (3mM) | 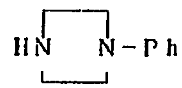 | 2 | 0.91 | 63.5 |
| 196 | 337 | 636 1.01g (3mM) | 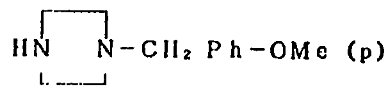 | 2.5 | 1.38 | 88.1 |
| 197 | 338 | 636 1.01g (3mM) | 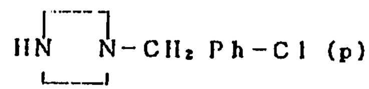 | 2 | 1.32 | 83.6 |
| 198 | 339 | 636 1.01g (3mM) | 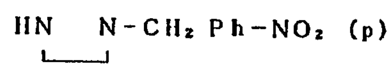 | 3 | 1.48 | 89.5 |
| 199 | 340 | 636 1.01g (3mM) | 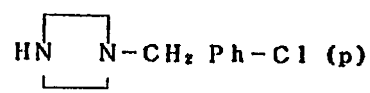 | 2 | 1.24 | |

Table 20 ( Continued )

| | | | | | | | |
|---|---|---|---|---|---|---|---|
| 200 | 276 | 637 | 1.09g (3mM) | 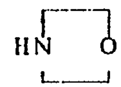 | 2 | 1.16 | 89.5 |
| 201 | 277 | 637 | 1.09g (3mM) | 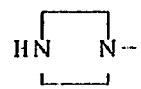 $-CH_2Ph-Cl$ (p) | 2 | 1.27 | 76.3 |
| 202 | 278 | 637 | 1.09g (3mM) | 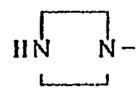 $-CH_2Ph-F$ (p) | 2 | 0.60 | 36.2 |
| 203 | 279 | 637 | 1.09g (3mM) | 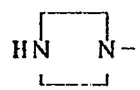 $-CH_2Ph-NO_2$ (p) | 2 | 1.35 | 79.6 |
| 204 | 280 | 638 | 1.17g (3mM) | 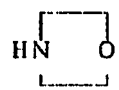 | 2 | 0.91 | 66.1 |
| 205 | 281 | 638 | 1.17g (3mM) | 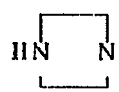 $-CH_2Ph-OMe$ (p) | 2 | 1.21 | 69.8 |

Table 20 ( Continued )

| | | | | | | | |
|---|---|---|---|---|---|---|---|
| 206 | 282 | 638 | 1. 17g (3mM) | 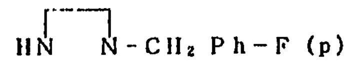 | 2 | 0. 97 | 55. 7 |
| 207 | 487 | 638 | 1. 17g (3mM) | 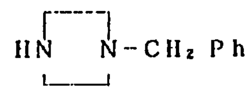 | 2 | 1. 43 | 87. 0 |
| 208 | 488 | 638 | 1. 17g (3mM) | 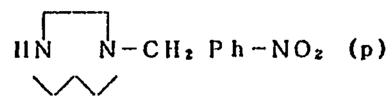 | 3 | 0. 89 | 48. 9 |
| 209 | 489 | 638 | 1. 17g (3mM) | 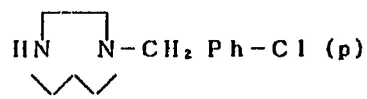 | 3 | 0. 92 | 51. 4 |
| 210 | 490 | 638 | 1. 17g (3mM) | 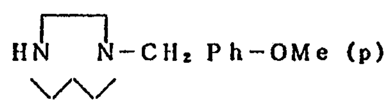 | 2. 5 | 0. 98 | 55. 2 |
| 211 | 283 | 639 | 1. 26g (3mM) | 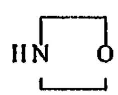 | 2 | 1. 22 | 83. 5 |

Table 20 ( Continued )

| | | | | | | | |
|---|---|---|---|---|---|---|---|
| 212 | 284 | 639 | 1.26g (3mM) | 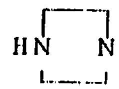-$CH_2$Ph-Cl (p) | 2 | 1.52 | 83.0 |
| 213 | 285 | 639 | 1.26g (3mM) | 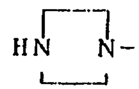-$CH_2$Ph-$NO_2$ (p) | 2 | 1.77 | 95.0 |
| 214 | 286 | 639 | 1.26g (3mM) | 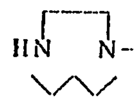-$CH_2$Ph-Cl (p) | 2 | 1.76 | 93.9 |

* ; Compound No.

Table 21 1-(2-(4'-acylpiperadinyl) ethyl)-3-ehtyl-5, 6-dimethyl-2-oxo-1, 2-dihydropyrazine

| Example | Product* | Acylation Method | Acylating agent | Reaction time | Elution solvent ratio | Yield (g) | Yield (%) |
|---|---|---|---|---|---|---|---|
| 216 | 455 | acid chloride | $ClCO-CH_2Ph$ | 1h | 100:1 | 0.93 | 81.2 |
| 217 | 457 | acid anhydride | 2-thienylchloride | 4 | 100:1 | 1.35 | 87.0 |
| 218 | 459 | acid anhydride | $HOOC-CH_2Ph-NO_2$ (p) | 5 | 200:1 | 0.98 | 57.4 |
| 219 | 465 | acid chloride | $ClSO_2-Ph-Cl$ (p) | 1 | 200:1 | 1.23 | 93.5 |
| 220 | 456 | acid anhydride | $HOOC-Ph-Cl$ (p) | 2 | 100:1 | 1.49 | 92.5 |
| 221 | 458 | acid anhydride | $HOOC-Ph-NO_2$ (p) | 2 | 100:1 | 1.59 | 96.2 |
| 222 | 460 | acid chloride | $HOOC-Ph-OMe$ (p) | 1 | 100:1 | 1.07 | 89.6 |
| 223 | 461 | acid anhydride | $HOOC-Ph-OMe_2$ (2, 3) | 2 | 100:1 | 1.56 | 91.1 |

Table 21 ( Continued )

| | | | | | | | |
|---|---|---|---|---|---|---|---|
| 224 | 462 | acid anhydride | $HOOC-Ph-OMe_2$ (2, 4) | 2 | 200:1 | 1.51 | 88.2 |
| 225 | 463 | acid anhydride | $HOOC-Ph-OMe_2$ (3, 4) | 4 | 100:1 | 1.55 | 90.5 |
| 226 | 464 | acid anhydride | $HOOC-Ph-OMe_3$ (3, 4, 5) | 3 | 100:1 | 1.40 | 99.0 |

*; Compound No.

Table 22 1 - (2 -substituted ethyl)-3-benzyl - 2 -oxo - 1, 2, 5, 6, 7, 8 -hexahydro quinoxaline

| Compound | Product * | Base | Starting material * | Heat time ( hr ) | Elution solvent ratio | Yield ( g ) | Yield ( % ) |
|---|---|---|---|---|---|---|---|
| 229 | 470 | 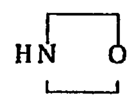 | 643 | 3 h | 100 : 1 | 0. 67 | 52. 6 |
| 230 | 471 | 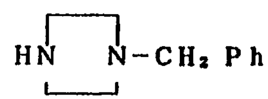 | 643 | 3 | 100 : 1 | 1. 01 | 62. 3 |
| 231 | 472 | 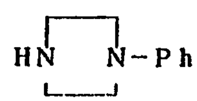 | 643 | 3 | 200 : 1 | 0. 82 | 54. 7 |
| 232 | 473 | 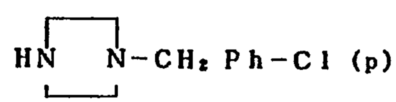 | 643 | 3 | 200 : 1 | 1. 10 | 66. 0 |

Table 22 ( Continued )

| | | | | | | | |
|---|---|---|---|---|---|---|---|
| 233 | 474 | 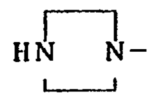 $-CH_2Ph-OMe$ (p) | 643 | 2.5 | 100:1 | 0.93 | 56.3 |
| 234 | 475 | 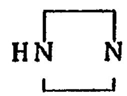 $-CH_2Ph-F$ (p) | 643 | 2.75 | 100:1 | 1.15 | 71.4 |

*; Compound No.

TABLE 23 1-(2-substituted ethyl)-3-(2-phenylethyl)-2-oxo-1, 2, 5, 6, 7, 8-hexahydro quinoxaline

| Example | product* | Base | Starting material* | Heat time ( hr ) | Elution solvent ratio | Yield ( g ) | Yield ( % ) |
|---|---|---|---|---|---|---|---|
| 237 | 476 | 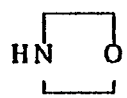 | 645 | 2h | 100:1 | 1.07 | 83.3 |
| 238 | 477 | 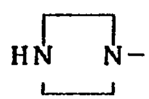 $-CH_2Ph$ | 645 | 2 | 100:1 | 1.35 | 84.6 |
| 239 | 478 | 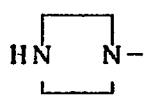 $-Ph$ | 645 | 2 | 200:1 | 1.01 | 65.2 |

Table 23 ( Continued )

| | | | | | | | |
|---|---|---|---|---|---|---|---|
| 240 | 479 | 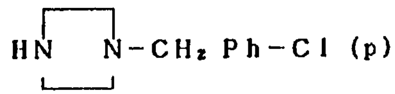 | 645 | 2. 5 | 100:1 | 1. 41 | 82. 0 |
| 241 | 480 | 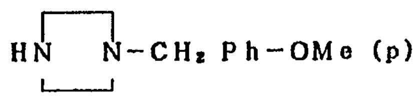 | 645 | 2. 5 | 100:1 | 0. 98 | 57. 6 |

*; Compound No.

Table 24 1-(2-substituted ethyl)-3-(2-phenylethyl)-2-oxo-1, 2, 5, 6, 7, 8-hexahydro quinoxaline

| Example | Product* | Base | Starting material* | Heat time ( hr ) | Elution solvent ratio | Yield ( g ) | Yield ( % ) |
|---|---|---|---|---|---|---|---|
| 243 | 481 | 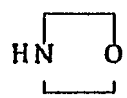 | 646 | 2. 5h | 100:1 | 0. 98 | 62. 6 |
| 244 | 482 | 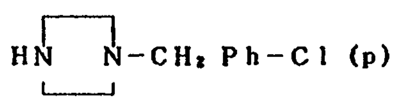 | 646 | 2. 5 | 100:1 | 1. 54 | 74. 3 |
| 245 | 483 | 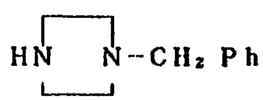 | 646 | 2 | 100:1 | 1. 67 | 87. 9 |

Table 24 ( Continued )

| | | | | | | | |
|---|---|---|---|---|---|---|---|
| 246 | 484 | 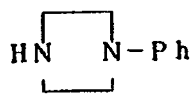 | 646 | 2. 5 | 200:1 | 1. 29 | 70. 5 |
| 247 | 485 | 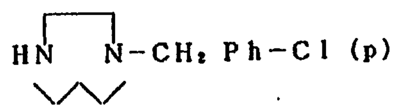 | 646 | 2 | 100:1 | 1. 25 | 58. 3 |
| 248 | 486 | 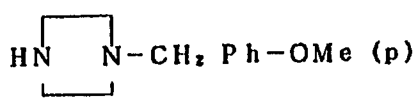 | 646 | 3 | 100:1 | 1. 78 | 86. 8 |

* ; Compound No.

Table 25 2-hydroxy -3-alkyl -5, 6, 7, 8-tetrahydro quinoxaline

$R_1-CHCONH_2$ (with $NH_2 \cdot HCl$ on the CH) +

α-amino acidamide hydrochloride

| Ref. Exa. | Product ($R_1$) | Yield (g) | Yield (%) | NMR ($CDCl_3$) δ (ppm) | Mass |
|---|---|---|---|---|---|
| 3 | Bu | 7.49 | 73.0 | 0.94 (t, 3H, buthyl 4-$CH_3$), 1.24~2.00 (m, buthyl 2-$CH_2$, 3-$CH_2$, quinoxaline 6-$H_2$, 7-$H_2$), 2.55~2.85 (m, 6H, buthyl 1-$CH_2$, quinoxaline 5-$H_2$, 8-$H_2$), 12.99 (br, s, 1H, OH) | 207 ($M^+$+1) |
| 4 | iso-Bu | 6.58 | 64.0 | 0.96 (d, 6H, isobuthyl 2×$CH_3$), 1.6~2.0 (m, 4H, 6-$H_2$, 7-$H_2$), 2.0~2.4 (isobuthyl CH), 2.5~2.8 (m, 6H, 5-$H_2$, 8-$H_2$) | 207 ($M^+$+1) |
| 5 | -$(CH_2)_4CH_3$ | 9.03 | 82.0 | 0.90 (t, 3H, J≒6.3), 1.2~1.5 (m, 4H), 1.5~1.9 (m, 6H), 2.5~2.9 (m, 6H) | 221 ($M^+$+1)<br>164 |
| 6 | -$(CH_2)_5CH_3$ | 7.81 | 66.7 | 0.88 (t, 3H, J≒7.9), 1.1~1.5 (m, 6H), 1.5~2.0 (m, 6H), 2.4~2.9 (m, 6H) | 235 ($M^+$+1) |

| | | | | | |
|---|---|---|---|---|---|
| 7 | $-(CH_2)_5CH_3$ | 9.33 | 75.0 | 0.88 (t, 3H, J≒7.4)、1.1~1.5 (m, 8H)、1.5~2.0 (m, 6H)、2.5~2.9 (m, 6H) | 249 ($M^+$+1) |
| 8 | $-(CH_2)_7CH_3$ | 11.67 | 89.1 | 0.88 (t, 3H, J≒7.5)、1.1~1.5 (m, 10H)、1.5~2.0 (m, 6H)、2.5~2.9 (m, 6H) | 263 ($M^+$+1) |
| 9 | $-(CH_2)_8CH_3$ | 12.73 | 92.2 | 0.88 (t, 3H, J≒6.2)、1.1~1.5 (m, 12H)、1.5~2.0 (m, 6H)、2.5~2.8 (m, 6H) | 277 ($M^+$+1)<br>164 |
| 10 | $-(CH_2)_9CH_3$ | 10.46 | 72.4 | 0.88 (t, 3H, J≒6.0)、1.2~2.0 (m, 20H)、2.5~2.9 (m, 6H)、12.7 (br.s, 1H) | 291 ($M^+$+1) |
| 11 | $-(CH_2)_{11}CH_3$ | 14.00 | 88.1 | 0.88 (t, 3H, J≒6)、1.1~1.9 (m, 24H)、2.4~2.8 (m, 6H) | 319 ($M^+$+1)<br>164 |
| 12 | $-(CH_2)_{13}CH_3$ | 16.58 | 95.8 | 0.88 (t, 3H, J≒6)、1.1~1.9 (m, 28H)、2.4~2.8 (m, 6H) | 347 ($M^+$+1)<br>164 |
| 13 | $-(CH_2)_{15}CH_3$ | 18.55 | 99.2 | 0.88 (t, 3H, J≒6)、1.1~1.9 (m, 32H)、2.5~2.8 (m, 6H) | 375 ($M^+$+1)<br>164 |

Table 26 2-hydroxy-3-alkyl-5, 6-diethylpyrazine

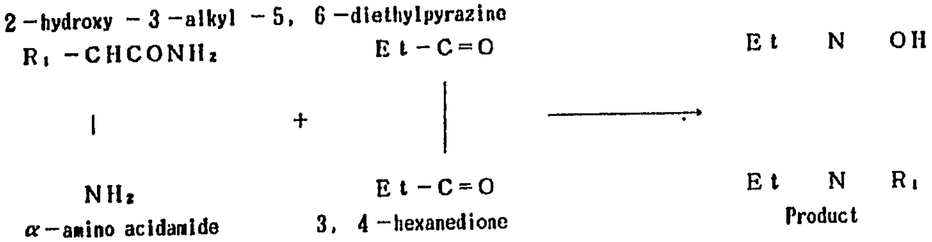

α-amino acidamide 3, 4-hexanedione Product

| Ref. Exa. | Product ($R_1$) | Yield (g) | Yield (%) | NMR ($CDCl_3$) δ (ppm) | Mass |
|---|---|---|---|---|---|
| 16 | Et | 14.4 | 40 | 1.1~1.4 (m, 9H), 2.3~3.0 (m, 6H), 13.2 (br. s, 1H) | 167 |
| 17 | Pro | 16.5 | 43 | 1.00 (t, 3H), 1.19 (t, 3H), 1.28 (t, 3H), 1.5~1.9 (m, 2H), 2.47 (q, 2H), 2.54 (q, 2H), 2.76 (q, 2H), 13.1 (br. s, 1H) | 181 |
| 18 | iso-Pro | 18.2 | 47 | 1.0~1.4 (m, 12H), 2.56 (q, 2H), 2.58 (q, 2H), 3.40 (sept, 1H), 12.9 (br. s, 1H) | 195 |
| 19 | Bu | 17.5 | 42 | 0.95 (t, 3H), 1.19 (t, 3H), 1.27 (t, 3H), 1.2~1.9 (m, 4H), 2.57 (q, 2H×2), 2.73 (t, 3H), 12.9 (br. s, 1H) | 209 |

| | | | | | |
|---|---|---|---|---|---|
| 20 | iso-Bu | 16.5 | 40 | 0.96 (t, 3H)、1.19 (t, 3H)、1.28 (t, 3H)、1.9~2.4 (m, H)、2.41 (q, 2H)、2.58 (q, 2H)、2.66 (d, 2H)、13.0 (br. s, 1H) | 209 |
| 21 | sec-Bu | 17.5 | 42 | 0.88 (t, 3H×2)、1.0~1.4 (m, 9H)、1.4~2.0 (m, 2H)、2.46 (q, 2H)、2.57 (q, 2H)、12.8 (br. s, 1H) | 209 |
| 22 | $-CH_2Ph$ | 27.9 | 57.6 | 1.19 (t, 3H, J≒7.6)、1.23 (t, 3H, J≒7.6)、2.50 (q, 2H, J≒7.6)、2.53 (q, 2H, J≒7.6)、4.07 (s, 2H)、7.0~7.4 (m, 5H)、13.2 (br. s, 1H) | 243 |
| 23 | $-CH_2Ph-Cl$ (p) | 27.4 | 49.6 | 1.19 (t, 3H, J≒7.7)、1.23 (t, 3H, J≒7.7)、2.53 (q, 2H, J≒7.7)、2.57 (q, 2H, J≒7.7)、4.03 (s, 2H)、7.0~7.4 (m, 4H)、13.3 (br. s, 1H) | 279<br>277 |

**Claims**

1. A compound of the formula (I):

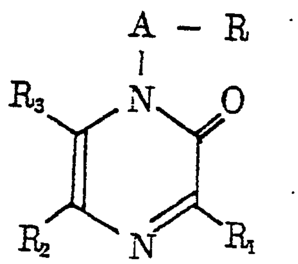

(I)

wherein R is hydroxyl, halogen, $C_{1-6}$ alkanoyloxy, $R_4$–carbamoyloxy, arylthio, 1-methyltetrazole-5-yl-thio, 1-imidazolyl, morpholino,

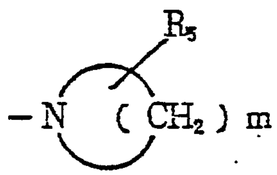 , 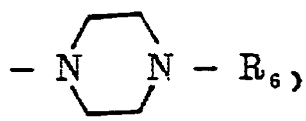 , 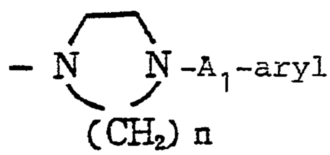

or 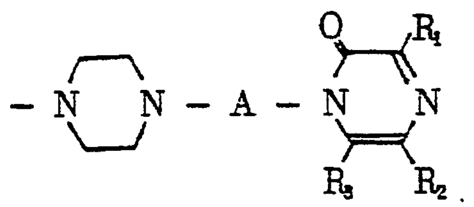 .

in which $R_4$ is $C_{1-6}$ alkyl or aryl, $R_5$ is hydrogen, $C_{1-6}$ alkyl or aryl, $R_6$ is hydrogen, $C_{1-4}$ alkyl, hydroxy-$C_{1-4}$ alkyl, aryl, aryl-$C_{2-4}$ alkanoyl, arylcarbonyl, arylsulfonyl or thienyl-$C_{2-4}$ alkanoyl, A and $A_1$ are $C_{1-3}$ alkylene, m is an integer of from 4 to 6, n is the integer 2 or 3; $R_1$ is alkyl, aryl-$C_{1-3}$ alkyl or aryl; and $R_2$ and $R_3$ are each $C_{1-3}$ alkyl or together constitute tetramethylene; and pharmacologically acceptable non-toxic salts thereof; wherein aryl means phenyl or unsubstituted by $C_{1-3}$ alkyl, halogen, nitro or lower alkoxy.

2. A compound according to claim 1, wherein $R_1$ is methyl, ethyl, phenyl, benzyl, p-chlorobenzyl or 2-phenylethyl.

3. A compound according to claim 1 or 2, wherein $R_2$ and $R_3$ are both methyl or are both ethyl.

4. A compound according to any one of the preceding claims, wherein A is $-CH_2CH_2-$.

5. A process for the preparation of a compound of formula (I) as defined in claim 1 wherein R is hydroxyl, or a pharmacologically acceptable non-toxic salt thereof, which process comprises reacting a compound of the formula (2)

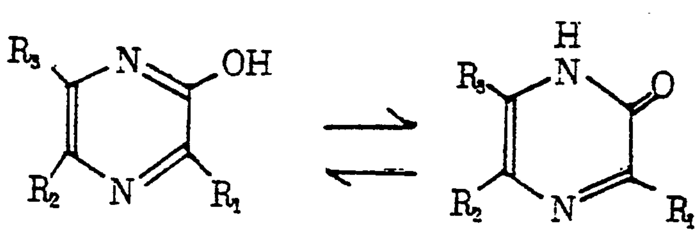

(2)

wherein $R_1$, $R_2$ and $R_3$ are as defined in claim 1, with a hydroxyalkyl halide of the formula:

X–A–OH

wherein X is halogen and A is as defined in claim 1, in an organic solvent and, if desired, converting the resultant compound of formula (I) wherein R is hydroxyl into a pharmacologically acceptable non-toxic salt thereof.

6. A process for the preparation of a compound of formula (I) as defined in claim 1 wherein R is $C_{1-6}$ alkanoyloxy, or a pharmacologically acceptable non-toxic salt thereof, which process comprises acylating a compound of formula (I) as defined in claim 1 wherein R is hydroxyl such as to convert the said hydroxyl into a $C_{1-6}$ alkanoyloxy group and, if desired, converting the resultant compound of formula (I) wherein R is $C_{1-6}$ alkanoyloxy into a pharmacologically acceptable non-toxic salt thereof.

7. A process for the preparation of a compound of formula (I) as defined in claim 1 wherein R is $R_4$–carbamoyloxy, $R_4$ being as defined in claim 1, or a pharmacologically acceptable non-toxic salt thereof, which process comprises reacting a compound of formula (I) as defined in claim 1 wherein R is hydroxyl with a $C_{1-6}$ alkyl isocyanate or aryl isocyanate and, if desired, converting the resultant compound of formula (I) wherein R is $R_4$– carbamoyloxy into a pharmacologically acceptable non-toxic salt thereof.

8. A process for the preparation of a compound of formula (I) as defined in claim 1 wherein R is halogen, or a pharmacologically acceptable non-toxic salt thereof, which process comprises halogenating a compound of formula (I) as defined in claim 1 wherein R is hydroxyl and, if desired, converting the resultant compound of formula (I) wherein R is halogen into a pharmacologically acceptable non-toxic salt thereof.

9. A process for the preparation of a compound of formula (I) as defined in claim 1 wherein R is arylthio or 1-methyltetrazole-5-yl-thio or a pharmacologically acceptable non-toxic salt thereof, which process comprises reacting a compound of formula (I) as defined in claim 1 wherein R is halogen with an alkali or alkaline earth metal salt of arylmercaptan or of 1-methyl-5-mercaptotetrazole in an organic solvent and, if desired, converting the resultant compound of formula (I) wherein R is arylthio or 1-methyltetrazole-5-yl-thio into a pharmacologically acceptable nontoxic salt thereof.

10. A process for the preparation of a compound of formula (I) as defined in claim 1 except R is not hydroxyl, halogen, $C_{1-6}$ alkanoyloxy, $R_4$-carbamoyloxy, arylthio or 1-methyltetrazole-5-yl-thio, or a pharmacologically acceptable non-toxic salt thereof, which process comprises reacting a compound of formula (I) as defined in claim 1 wherein R is halogen with an amine of the formula

R′–H

wherein R′ is 1-imidazolyl, morpholino,

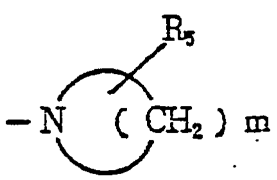 , 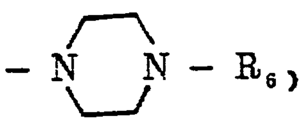 , 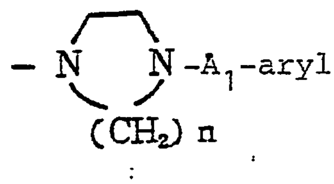

or 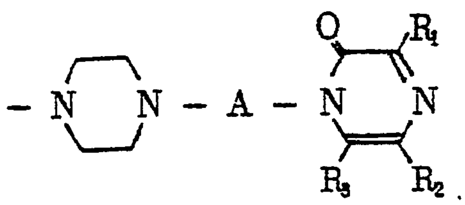

wherein $R_1$, $R_2$, $R_3$, $R_5$, $R_6$, A, $A_1$, m and n are as defined in claim 1 and, if desired, converting the resultant compound of formula (I) into a pharmacologically acceptable non-toxic salt thereof.

11. A process for the preparation of a compound of formula (I) as defined in claim 1 wherein R is

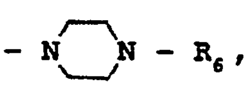 ,

in which $R_6$ is aryl-$C_{2-4}$ alkanoyl, arylcarbonyl, arylsulfonyl or thienyl-$C_{2-4}$ alkanoyl, or a pharmacologically acceptable non-toxic salt thereof, which process comprises arylating a compound (1f′) of the formula

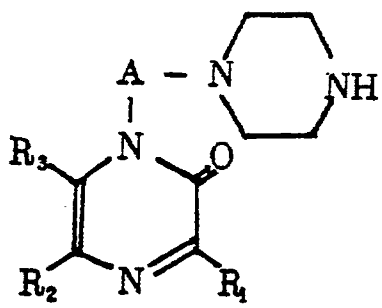

[1f']

wherein $R_1$, $R_2$, $R_3$ and A are as defined above, such as to convert the piperazinyl group thereof into a group of formula

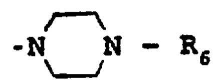

and, if desired, converting the resultant compound of formula (I) into a pharmacologically acceptable non-toxic salt thereof.

12. A pharmaceutical composition comprising as active ingredient a compound of formula (I) as defined in claim 1 or a pharmacologically acceptable non-toxic salt thereof, together with a pharmaceutically acceptable carrier or diluent.

**Patentansprüche**

1. Verbindung der Formel (I):

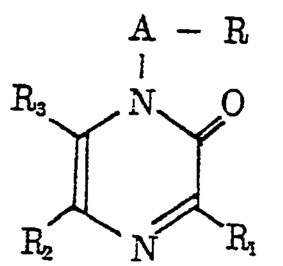

(I)

worin R Hydroxyl, Halogen, $C_{1-6}$-Alkanoyloxy, $R_4$-Carbamoyloxy, Arylmercapto, 1-Methyltetrazol-5-ylmercapto, 1-Imidazolyl, Morpholino,

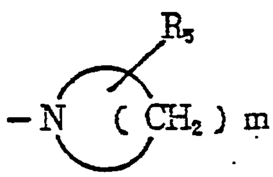 , 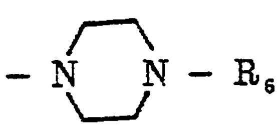 , 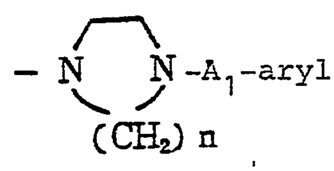

or 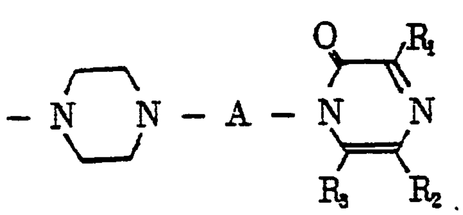

bedeutet, worin $R_4$ $C_{1-6}$-Alkyl oder Aryl ist, $R_5$ Wasserstoff, $C_{1-6}$-Alkyl oder Aryl ist, $R_6$ Wasserstoff, $C_{1-4}$-Alkyl, Hydroxy-$C_{1-4}$-alkyl, Aryl, Aryl-$C_{2-4}$-alkanoyl, Arylcarbonyl, Arylsulfonyl oder Thienyl-$C_{2-4}$-alkanoyl ist, A und $A_1$ $C_{1-3}$-Alkylen sind, m eine ganze Zahl von 4 bis 6 ist, n die ganze Zahl 2 oder 3 bedeutet; $R_1$ Alkyl, Aryl-$C_{1-3}$-alkyl oder Aryl ist; und jeder $R_2$ und $R_3$ $C_{1-3}$ Alkyl ist oder zusammen Tetramethylen bilden; und pharmakologisch annehmbare nicht toxische Salze davon; und worin Aryl unsubstituiertes oder durch $C_{1-3}$-Alkyl, Halogen, Nitro oder Niederalkoxy substituiertes Phenyl bedeutet.

2. Verbindung nach Anspruch 1, dadurch gekennzeichnet, daß $R_1$ Methyl, Ethyl, Phenyl, Benzyl, p-Chlorbenzyl oder 2-Phenylethyl ist.

3. Verbindung nach Anspruch 1 oder 2, dadurch gekennzeichnet, daß $R_2$ und $R_1$ beide Methyl oder beide Ethyl sind.

4. Verbindung nach einem der vorhergehenden Ansprüche, dadurch gekennzeichnet, daß A $-CH_2CH_2-$ ist.

5. Verfahren zur Herstellung einer Verbindung der wie im Anspruch 1 definierten Formel (I), worin R Hydroxy ist, oder eines pharmakologisch annehmbaren nicht toxischen Salzes davon, dadurch gekennzeichnet, daß man eine Verbindung der Formel (2)

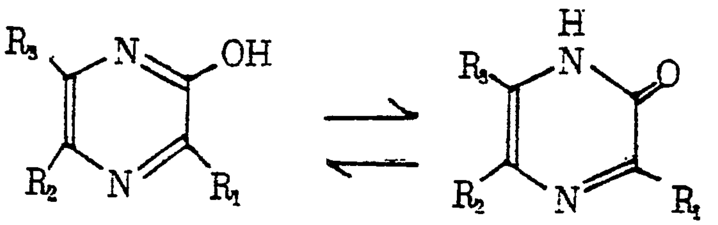 (2)

worin $R_1$, $R_2$ und $R_3$ die in Anspruch 1 angegebene Bedeutung besitzen, mit einem Hydroxyalkylhalogenid der Formel

X–A–OH

worin X Halogen ist und A die im Anspruch 1 angegebene Bedeutung besitzt, in einem organischen Lösungsmittel umsetzt, und wenn erwünscht, die erhaltene Verbindung der Formel (I), worin R Hydroxy ist, in ein pharmakologisch annehmbares nicht toxisches Salz davon überführt.

6. Verfahren zur Herstellung einer Verbindung der wie im Anspruch 1 definierten Formel (I), worin R $C_{1-6}$-Alkanoyloxy ist, oder eines pharmakologisch annehmbaren nicht toxischen Salzes davon, dadurch gekennzeichnet, daß man eine Verbindung der in Anspruch 1 definierten Formel (I), worin R Hydroxy ist, so acyliert, um die Hydroxygruppe in einer $C_{1-6}$-Alkanoyloxygruppe zu überführen, und wenn erwünscht, die erhaltene Verbindung der Formel (I), worin R $C_{1-6}$-Alkanoyloxy bedeutet, in ein pharmakologisch annehmbares nicht toxisches Salz davon überführt.

7. Verfahren zur Herstellung einer Verbindung der wie in Anspruch 1 definierten Formel (I), worin R $R_4$-Carbamoyloxy ist, und $R_4$ die in Anspruch 1 angegebene Bedeutung besitzt, oder eines pharmakologisch annehmbaren nicht toxischen Salzes davon, dadurch gekennzeichnet, daß man eine Verbindung der wie in Anspruch 1 definierten Formel (I), worin R Hydroxy ist, mit einem $C_{1-6}$-Alkylisocyanat oder Arylisocyanat umsetzt, und wenn erwünscht, die erhaltene Verbindung der Formel (I), worin R $R_4$-Carbamoyloxy ist, in ein pharmakologisch annehmbares nicht toxisches Salz davon überführt.

8. Verfahren zur Herstellung einer Verbindung der wie in Anspruch 1 definierten Formel (I), worin R Halogen ist, oder eines pharmakologisch annehmbaren nicht toxischen Salzes davon, dadurch gekennzeichnet, daß man eine Verbindung der wie in Anspruch 1 definierten Formel (I), worin R Hydroxy ist, halogeniert, und wenn erwünscht, die erhaltene Verbindung der Formel (I), worin R Halogen ist, in ein pharmakologisch annehmbares nicht toxisches Salz davon überführt.

9. Verfahren zur Herstellung einer Verbindung der wie in Anspruch 1 definierten Formel (I), worin R Arylmercapto oder 1-Methyltetrazol-5-yl-mercapto ist, oder eines pharmakologisch annehmbaren nicht toxischen Salzes davon, dadurch gekennzeichnet, daß man eine Verbindung der wie in Anspruch 1 definierten Formel (I), worin R Halogen ist, mit einem Alkali- oder Erdalkalimetallsalz von Arylmercaptan oder von 1-Methyl-5-mercaptotetrazol in einem organischen Lösungsmittel umsetzt, und, wenn erwünscht, die erhaltene Verbindung der Formel (I), worin R Arylmercapto oder 1-Methyltetrazol-5-yl-mercapto ist, in ein pharmakologisch annehmbares nicht toxisches Salz davon überführt.

10. Verfahren zur Herstellung einer Verbindung der wie in Anspruch 1 definierten Formel (I), mit der Ausnahme, daß R nicht Hydroxy, Halogen, $C_{1-6}$-Alkanoyloxy, $R_4$-Carbamoyloxy, Arylmercapto oder 1-Methyltetrazol-5-yl-mercapto ist, oder eines pharmakologisch annehmbaren, nicht toxischen Salzes davon, dadurch gekennzeichnet, daß man eine Verbindung der wie in Anspruch 1 definierten Formel (I), worin R Halogen ist, mit einem Amin der Formel R′–H umsetzt, worin R′ 1-Imidazolyl, Morpholino,

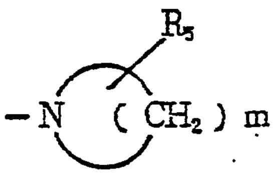, 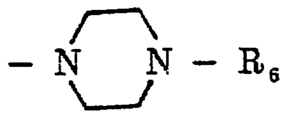, 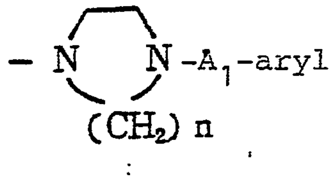,

or 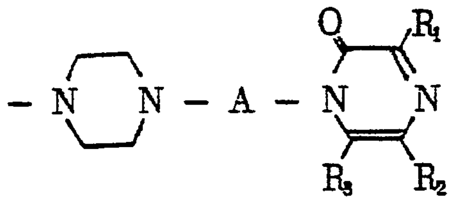.

ist, worin $R_1$, $R_2$, $R_3$, $R_5$, $R_6$, A, $A_1$, m und n die in Anspruch 1 angegebene Bedeutung besitzen, und wenn erwünscht, die erhaltene Verbindung der Formel (I) in ein pharmakologisch annehmbares nicht toxisches Salz davon überführt.

11. Verfahren zur Herstellung einer Verbindung der wie in Anspruch 1 definierten Formel (I), worin R

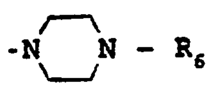

ist, worin $R_6$ Aryl-$C_{2-4}$-alkanyl, Arylcarbonyl, Arylsulfonyl oder Thienyl-$C_{1-4}$-alkanoyl bedeutet, oder eines pharmakologisch annehmbaren nicht toxischen Salzes davon, dadurch gekennzeichnet, daß man eine Verbindung (1f') der Formel

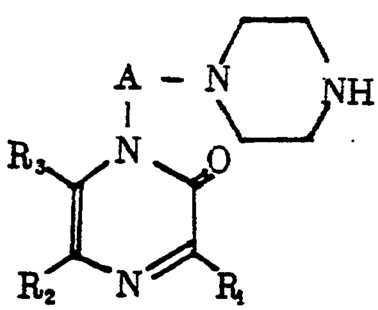

[1f']

worin $R_1$, $R_2$, $R_3$ und A die vorstehend angegebene Bedeutung besitzen, aryliert, um die Piperazinylgruppe davon in eine Gruppe der Formel

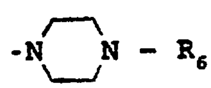

zu überführen, und, wenn erwünscht, die erhaltene Verbindung der Formel (I) in ein pharmakologisch annehmbares nicht toxisches Salz davon überführt.

12. Pharmazeutische Zusammensetzung, dadurch gekennzeichnet, daß sie als aktiven Bestandteil eine Verbindung der wie in Anspruch 1 definierten Formel (I) oder ein pharmakologisch annehmbares nicht toxisches Salz davon zusammen mit einem pharmazeutisch annehmbaren Träger oder Verdünnungsmittel enthält.

**Revendications**

1. Composé de la formule (1):

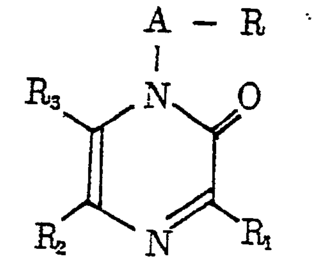

(I)

dans laquelle R est un hydroxyle, un atome d'halogène, un alcanoyloxy à 1 à 6 atomes de carbone, un carbamoyloxy-$R_4$, un arylthio, 1-méthyltétrazole-5-yl-thio, 1-imidazolyle, un morpholino,

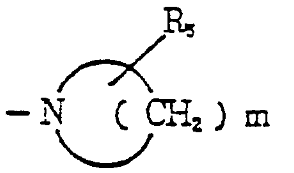
,
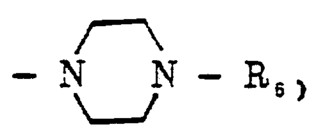
,
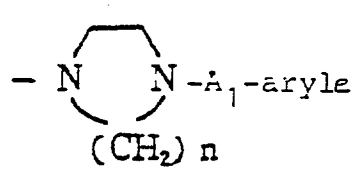

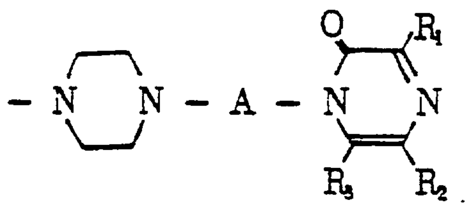

dans lesquelles $R_4$ est un alcoyle à 1 à 6 atomes de carbone ou un aryle, $R_5$ est un atome d'hydrogène, un alcoyle à 1 à 6 atomes de carbone ou un aryle, $R_6$ est un atome d'hydrogène ou l'un des groupes suivants: alcoyle à 1 à 4 atomes de carbone, hydroxy-alcoyle à 1 à 4 atomes de carbone, aryle, aryl-alcanoyle à 2 à 4 atomes de carbone, arylcarbonyle, arylsulfonyle ou thiényl-alcanoyle à 2 à 4 atomes de carbone, A et $A_1$ sont un alcoylène à 1 à 3 atomes de carbone, m est un nombre entier égal à 4, 5 ou 6, n est égal à 2 ou 3, $R_1$ est un alcoyle, un aryl-alcoyle à 1 à 3 atomes de carbone ou un aryle; et $R_2$ et $R_3$ sont chacun un alcoyle à 1 à 3 atomes de carbone ou constituent ensemble du tétraméthylène; et leurs sels non toxiques pharmaceutiquement acceptables, le terme "aryle" signifiant ici un phényle non substitué ou substitué par un alcoyle à 1 à 3 atomes de carbone, un halogène, un groupe nitro ou un alcoxy inférieur.

2. Composé selon la revendication 1, dans lequel $R_1$ est l'un des groupes suivants: méthyle, éthyle, phényle, benzyle, p-chlorobenzyle ou 2-phényléthyle.

3. Composé selon la revendication 1 ou 2, dans lequel $R_2$ et $R_3$ sont tous deux du méthyle ou tous deux de l'éthyle.

4. Composé selon l'une quelconque des revendications précédentes, dans lequel A est $-CH_2CH_2$.

5. Procédé pour préparer un composé de la formule (1) comme défini dans la revendication 1, dans lequel R est un hydroxyle ou un de ses sels non toxiques pharmaceutiquement acceptables, lequel procédé consiste à faire réagir un composé de la formule (2):

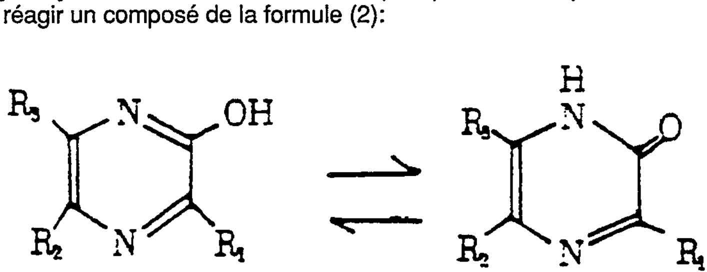

(2)

dans laquelle $R_1$, $R_2$ et $R_3$ sont comme définis dans la revendication 1 avec un halogénure d'hydroxyalcoyle de la formule:

X–A–OH

dans laquelle X est un halogène et A est comme défini dans la revendication 1, dans un solvant organique et, si on le désire, à convertir le composé résultant de la formule (1) dans laquelle R est un hydroxyle en un de ses sels non toxiques pharmaceutiquement acceptables.

6. Procédé pour préparer un composé de la formule (1) selon la revendication 1, dans lequel R est un alcanoyloxy à 1 à 6 atomes de carbone, ou l'un de ses sels non toxiques pharmaceutiquement acceptables, lequel procédé consiste à acyler un composé de la formule (1) selon la revendication 1, dans lequel R est un hydroxyle, de façon à convertir ce groupe hydroxyle en un groupe alcanoyloxy à 1 à 6 atomes de carbone et, si on le désire, à convertir le composé résultant de la formule (1) dans laquelle R est un alcanoyloxy à 1 à 6 atomes de carbone en un de ses sels non toxiques pharmaceutiquement acceptables.

7. Procédé pour préparer un composé de la formule (1) selon la revendication 1, dans lequel R est carbamoyloxy-$R_4$, $R_4$ étant comme défini dans la revendication 1, ou l'un de ses sels non toxiques pharmaceutiquement acceptables, lequel procédé consiste à faire réagir un composé de la formule (1) comme défini par la revendication 1, dans lequel R est un hydroxyle, avec un isocyanate d'alcoyle à 1 à 6 atomes de carbone ou un isocyanate d'aryle et, si on le désire, à convertir le composé résultant de la formule (1), dans lequel R est carbamoyloxy-$R_4$ en l'un des ses sels non toxiques pharmaceutiquement acceptables.

8. Procédé pour préparer un composé de la formule (1) tel que défini dans la revendication 1, dans lequel R est un halogène, ou l'un de ses sels non toxiques pharmaceutiquement acceptables, lequel procédé consiste à halogéner un composé de la formule (1) tel que défini dans la revendication 1, dans lequel R est un groupe hydroxyle et, si on le désire, à convertir le composé résultant de la formule (1), dans lequel R est un atome d'halogène, en l'un de ses sels non toxiques pharmaceutiquement acceptables.

9. Procédé pour préparer un composé de la formule (1), tel que défini dans la revendication 1, dans lequel R est un groupe arylthio ou un groupe 1-méthyltétrazole-5-yl-thio, ou l'un de ses sels non toxiques pharmaceutiquement acceptables, lequel procédé consiste à faire réagir un composé de la formule (1) comme défini dans la revendication 1, dans lequel R est un halogène, avec un sel de métal alcalin ou de métal alcalino-terreux d'arylmercaptan ou de 1-méthyl-5-mercaptotétrazole dans un solvant organique et, si on le désire, à convertir le composé résultant de la formule (1), dans lequel R est un groupe arylthio ou un groupe 1méthyl-tétrazole-5-yl-thio, en l'un de ses sels non toxiques pharmaceutiquement acceptables.

10. Procédé pour préparer un composé de la formule (1), tel que défini dans la revendication 1, sauf que R n'est pas un groupe hydroxyle, un atome d'halogène, un alcanoyloxy à 1 à 6 atomes de carbone, un carbamoyloxy-$R_4$, un groupe arylthio ou un groupe 1-méthyl-tétrazole-5-yl-thio, ou l'un de ses sels non toxiques pharmaceutiquement acceptables, lequel procédé consiste à faire réagir un composé de la formule (1) tel que défini dans la revendication 1, dans lequel R est un halogène, avec une amine de la formule:

R'–H

dans laquelle R' est 1-imidazolyle, morpholino,

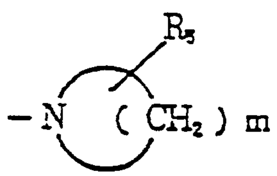 , 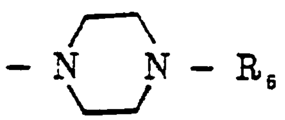 , 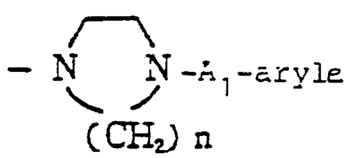

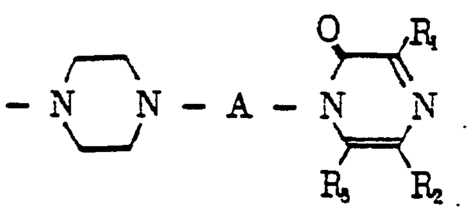 .

dans lesquelles $R_1$, $R_2$, $R_3$, $R_5$, $R_6$, A, $A_1$, met n sont tels que définis dans la revendication 1 et, si on le désire, à convertir le composé résultant de la formule (1) en l'un de ses sels non toxiques pharmaceutiquement acceptables.

11. Procédé pour préparer un composé de la formule (1) tel que défini dans la revendication 1, dans lequel R est

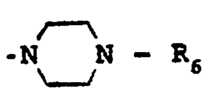

dans lequel $R_6$ est l'un des groupes suivants: aryl-alcanoyle à 2 à 4 atomes de carbone, arylcarbonyle, arylsulfonyle ou thiényl-alcanoyle à 2 à 4 atomes de carbone, ou l'un de ses sels non toxiques pharmaceutiquement acceptables, lequel procédé consiste à aryler un composé (1f') de la formule:

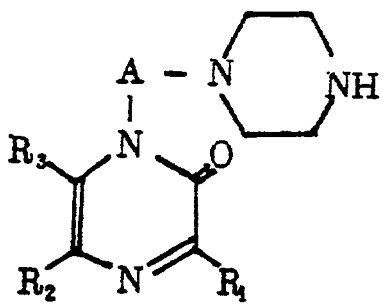

[1f']

dans laquelle $R_1$, $R_2$, $R_3$ et A sont comme définis ci-dessus, de façon à convertir son groupe pipérazinyle en un groupe de la formule:

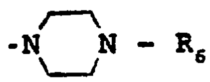

et, si on le désire, à convertir le composé résultant de la formule (1) en l'un de ses sels non toxiques pharmaceutiquement acceptables.

12. Composition pharmaceutique comprenant comme ingrédient actif un composé de la formule (1) tel que défini dans la revendication 1 ou l'un de ses sels non toxiques pharmaceutiquement acceptables, en combinaison avec un véhicule ou un diluant pharmaceutiquement acceptable.